(19) Europäisches Patentamt
European Patent Office
Office européen des brevets

(11) Veröffentlichungsnummer: **0 000 869**
**A1**

(12) # EUROPÄISCHE PATENTANMELDUNG

(21) Anmeldenummer: **78100431.2**

(22) Anmeldetag: **19.07.78**

(51) Int. Cl.[2]: **C 07 D 401/14,** A 61 K 31/495 // (C07D401/14, 241/18, 217/24, 211/58)

(30) Priorität: **27.07.77 LU 77856**

(43) Veröffentlichungstag der Anmeldung: **07.03.79 Patentblatt 79/5**

(84) Benannte Vertragsstaaten: **BE CH DE FR GB NL SE**

(71) Anmelder: **CIBA-GEIGY AG, Patentabteilung Postfach, CH-4002 Basel (CH)**

(72) Erfinder: **Schröter, Herbert, Dr., Arlsdörferstrasse 16, CH-4414 Füllinsdorf (CH)**
Erfinder: **Eichenberger, Kurt, Dr., Neubergweg 36, CH-4106 Therwil (CH)**
Erfinder: **Kühnis, Hans, Dr., Lange Gasse 26, CH-4052 Basel (CH)**
Erfinder: **Egli, Christian, Dr., Auf Egg, CH-4465 Magden (CH)**
Erfinder: **Schier, Oswald, Dr., Amselstrasse 15, CH-4104 Oberwil (CH)**
Erfinder: **Ostermayer, Franz, Dr., Am Hang 5, CH-4125 Riehen (CH)**

(54) **Neue Piperidin-Derivate, Verfahren zu ihrer Herstellung und pharmazeutische Präparate diese enthaltend.**

(57) Piperidinderivate der Formel I,

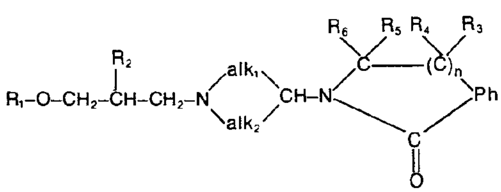

(I)

worin $R_1$ einen gegebenenfalls substituierten Heteroarylrest bedeutet, $alk_1$ und $alk_2$ unabhängig voneinander niedere Alkylenreste sind, die jeweils das mit ihnen verbundene Stickstoffatom und die mit ihnen verbundene Methingruppe durch 2 Kohlenstoffatome trennen, $R_2$ eine gegebenenfalls acylierte Hydroxylgruppe bedeutet, Ph einen gegebenenfalls substituierten o-Phenylenrest bedeutet, n = 0 oder 1 bedeutet, $R_3$ ein Wasserstoffatom, einen niederen Alkylrest oder die Hydroxylgruppe und $R_5$ einen niederen Alkylrest oder ein Wasserstoffatom bedeutet oder $R_3$ und $R_5$ zusammen für eine zweite Bindung stehen und $R_4$ und $R_6$ je ein Wasserstoffatom bedeuten, oder $R_4$ zusammen mit $R_3$ für eine Oxogruppe steht, $R_5$ einen niederen Alkylrest oder ein Wasserstoffatom und $R_6$ ein Wasserstoffatom bedeutet, oder $R_6$ zusammen mit $R_5$ für eine Oxogruppe steht, $R_3$ ein Wasserstoffatom, einen niederen Alkylrest oder die Hydroxylgruppe bedeutet und $R_4$ für ein Wasserstoffatom steht, und deren Salze, sowie Verfahren zur Herstellung dieser Verbindungen und pharmazeutische Präparate enthaltend diese Verbindungen.

EP 0 000 869 A1

Case 4-11272/+

BEZEICHNUNG GEÄNDERT
siehe Titelseite

Neue Piperidin-Derivate und Verfahren zu ihrer Herstellung

Die Erfindung betrifft neue Piperidinderivate der Formel I,

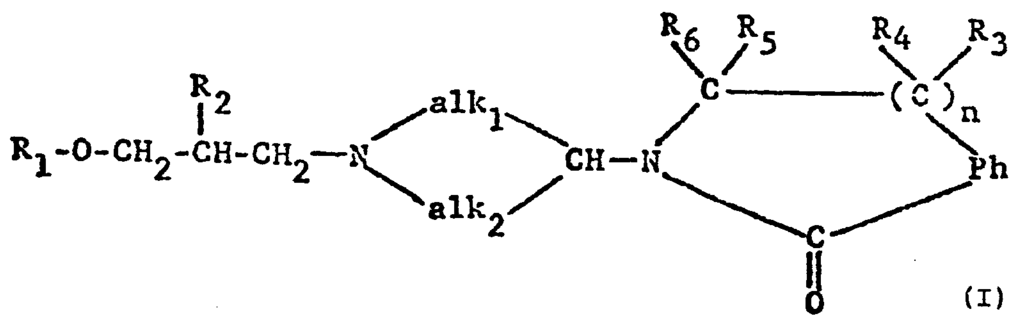

(I)

worin $R_1$ einen gegebenenfalls substituierten Heteroarylrest bedeutet, $alk_1$ und $alk_2$ unabhängig voneinander niedere Alkylenreste sind, die jeweils das mit ihnen verbundene Stickstoffatom und die mit ihnen verbundene Methingruppe durch 2 Kohlenstoffatome trennen, $R_2$ eine gegebenenfalls acylierte Hydroxylgruppe bedeutet, Ph einen gegebenenfalls substituierten o-Phenylenrest bedeutet, n = 0 oder 1 bedeutet, $R_3$ ein Wasserstoffatom, einen niederen Alkylrest oder die Hydroxylgruppe und $R_5$ einen niederen Alkylrest oder ein Wasserstoffatom bedeutet oder $R_3$ und $R_5$ zusammen für eine zweite Bindung stehen und $R_4$ und $R_6$ je ein Wasserstoff-

atom bedeuten, oder $R_4$ zusammen mit $R_3$ für eine Oxogruppe steht, $R_5$ einen niederen Alkylrest oder ein Wasserstoffatom und $R_6$ ein Wasserstoffatom bedeutet, oder $R_6$ zusammen mit $R_5$ für eine Oxogruppe steht, $R_3$ ein Wasserstoffatom, einen niederen Alkylrest oder die Hydroxylgruppe bedeutet und $R_4$ für ein Wasserstoffatom steht, und deren Salze, sowie Verfahren zur Herstellung dieser Verbindungen und pharmazeutische Präparate enthaltend diese Verbindungen.

Wenn n = 1 ist, betrifft die Erfindung Verbindungen der Formel

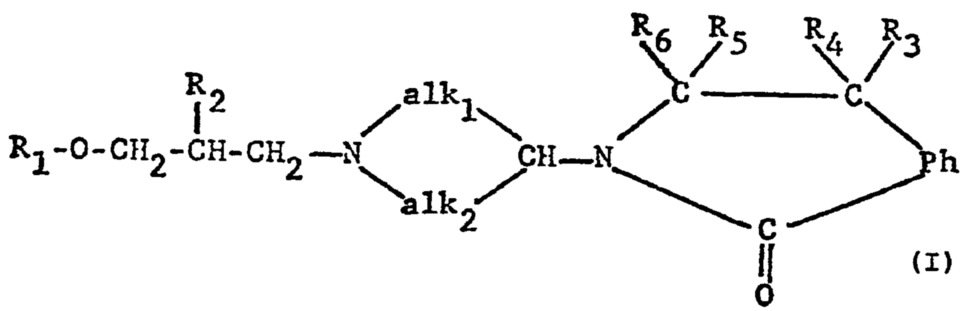

(I)

und, wenn n = 0 ist, betrifft die Erfindung Verbindungen der Formel

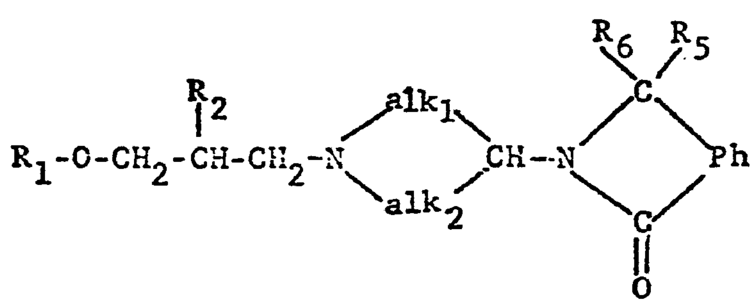

(I)

worin in den oberen Formeln $R_1$, $R_2$, $R_3$, $R_4$, $R_5$, $R_6$, Ph, $alk_1$ und $alk_2$ die vorher angegebenen Bedeutungen haben.

Wo nichts anderes angegeben, sind niedere Reste solche Reste, die nicht mehr als 7 Kohlenstoffatome und vorzugsweise bis zu 4 Kohlenstoffatome enthalten.

Ein Heteroarylrest $R_1$ ist ein gegebenenfalls substituierter

heterocyclischer, vorzugsweise monocyclischer, sowie bicyclischer Heteroarylrest, in erster Linie ein gegebenenfalls, z.B. einfach, zweifach oder mehrfach, substituierter, vorzugsweise monocyclischer Azaarylrest mit 5 bis 6 Ringgliedern und 1 bis 2 Ringstickstoffatomen, wie gegebenenfalls substituiertes Pyridyl, z.B. 2-, 3- oder 4-Pyridyl, Imidazolyl, z.B. 2-Imidazolyl, Pyrimidinyl, z.B. 2- oder 4-Pyrimidinyl, Pyridazinyl, z.B. 2-Pyridazinyl, oder Pyrazinyl, z.B. 2-Pyrazinyl, ferner ein gegebenenfalls substituierter bicyclischer Azaarylrest, insbesondere Benzoazaarylrest, mit 5 bis 6 Ringgliedern und 1 bis 2 Ringstickstoffatomen im Azaarylrest, wie gegebenenfalls substituiertes Indolyl, z.B. 4-Indolyl, Chinolinyl, z.B. 4-Chinolinyl, oder Isochinolinyl, z.B. 1-Isochinolinyl. Substituenten eines Heteroarylrestes sind u.a. gegebenenfalls substituierte aliphatische Kohlenwasserstoffreste, gegebenenfalls veräthertes oder verestertes Hydroxy oder Mercapto, Acyl, Nitro oder gegebenenfalls substituiertes Amino.

Niedere Alkylenreste $alk_1$ bzw. $alk_2$ sind beispielsweise 2,3-Butylenreste, 1,2-Butylenreste, 1,1-Dimethyl-1,2-äthylenreste, oder vorzugsweise 1,2-Propylenreste oder insbesondere 1,2-Aethylenreste.

Der o-Phenylenrest Ph kann einen, zwei oder mehr Substituenten tragen; er enthält vorzugsweise aber nicht mehr als zwei Substituenten. Als Substituenten des o-Phenylenrestes kommen insbesondere in Betracht: niedere Alkylreste, niedere Alkoxygruppen, Halogenatome, Trifluoromethylgruppen, Hydroxylgruppen sowie in zweiter Linie auch Acylaminogruppen, Nitrogruppen und Aminogruppen.

Eine gegebenenfalls acylierte Hydroxylgruppe $R_2$ ist beispielsweise eine Niederalkanoyloxygruppe, wie z.B. eine Acetoxy, Propionyloxy- oder Butyryloxygruppe oder vorzugsweise die Pivaloyloxygruppe, oder vor allem eine freie

Hydroxygruppe.

Gegebenenfalls substituierte aliphatische Kohlenwasserstoffreste als Substituenten eines Heteroarylrestes $R_1$ sind entsprechendes Niederalkyl, sowie Niederalkenyl oder Niederalkinyl, wobei Substituenten von Niederalkyl, in erster Linie gegebenenfalls veräthertes oder verestertes Hydroxy oder Mercapto, Acyl oder gegebenenfalls substituiertes Amino sind.

Niedere Alkylreste sind beispielsweise Methyl-, Aethyl-, n-Propyl- oder Isopropylreste, oder geradkettige oder verzweigte Butyl-, Pentyl- oder Hexylreste, die in beliebiger Stellung gebunden sein können.

Niedere Alkenylreste sind insbesondere Allyl- oder Methallylreste, und als niederer Alkinylrest kommt vor allem der Propargylrest in Betracht.

Veräthertes Hydroxy ist insbesondere Niederalkoxy oder Phenylniederalkoxy, ferner Niederalkenyloxy oder Niederalkinyloxy, sowie Hydroxyniederalkoxy, Niederalkoxyniederalkoxy, Niederalkylthioniederalkoxy oder Niederalkanoylniederalkoxy, während verestertes Hydroxy insbesondere Halogen, ferner Niederalkanoyloxy darstellt.

Veräthertes Mercapto ist in erster Linie Niederalkylthio, während verestertes Mercapto z.B. Niederalkanoylthio ist.

Acyl ist vorzugsweise der entsprechende Rest einer organischen Carbonsäure und steht z.B. für Aroyl oder für Niederalkanoyl. Acyl steht ebenfalls für den entsprechenden Rest eines Kohlensäurehalbderivates, wie für Niederalkoxycarbonyl oder gegebenenfalls substituiertes Carbamoyl. Ein Acylrest im weitesten Sinne der Definition ist auch Cyan. Bevorzugte Acylreste sind insbesondere Benzoyl oder Acetyl.

Gegebenenfalls substituiertes Amino ist Acylamino, insbesondere Niederalkanoylamino oder Niederalkoxycarbonylamino, ferner gegebenenfalls substituiertes Ureido. Substituiertes Amino ist ebenfalls Niederalkylamino der Diniederalkylamino, sowie Niederalkylenamino, Niederoxaalkylenamino der Niederazaalkylenamino, wobei im letzterem der Azastickstoff vorzugsweise,z.B. durch Niederalkyl, substituiert ist.

Niederalkoxy ist z.B. Methoxy, Aethoxy, n-Propyloxy, Isopropyloxy, n-Butyloxy oder Isobutyloxy.

Phenylniederalkoxy ist z.B.Benzyloxy oder 1- oder 2-Phenyläthoxy.

Hydroxyniederalkoxy ist z.B. 2-Hydroxyäthoxy, ferner 2- oder 3-Hydroxypropyloxy.

Niederalkoxyniederalkoxy ist u.a. Niederalkoxymethoxy oder 1- und insbesondere 2-Niederalkoxy-äthoxy, z.B. Methoxymethoxy, 2-Methoxy-äthoxy oder 2-Aethoxy-äthoxy.

Niederalkylthioniederalkoxy ist insbesondere Niederalkylthiomethoxy oder 1- und in erster Linie 2-Niederalkylthioäthoxy, z.B. 2-Methylthio-äthoxy oder 2-Aethylthioäthoxy.

Niederalkanoylniederalkoxy ist insbesondere Acetonyloxy.

Niederalkenyloxy ist z.B. Allyloxy, 2- oder 3-Methallyloxy oder 3,3-Dimethylallyloxy.

Niederalkinyloxy ist insbesondere Propargyloxy.

Halogen ist vorzugsweise Halogen mit Atomnummer bis zu 35, d.h. Fluor, Chlor oder Brom.

Niederalkanoyloxy steht z.B. für Acetyloxy, Propionyloxy oder Pivaloyloxy.

Niederalkylthio ist z.B. Methylthio, Aethylthio, n-Propylthio oder Isopropylthio.

Niederalkanoylthio ist u.a. Acetylthio oder Propionylthio.

Niederalkanoyl ist z.B. Acetyl, Propionyl oder Butyryl.

Niederalkoxycarbonyl ist z.B. Methoxycarbonyl oder Aethoxycarbonyl.

Gegebenenfalls substituiertes Carbamoyl ist z.B. Carbamoyl, oder N-Niederalkyl- oder N,N-Diniederalkylcarbamoyl, wie N-Methyl-carbamoyl, N,N-Dimethylcarbamoyl, N-Aethylcarbamoyl oder N,N-Diäthylcarbamoyl.

Niederalkanoylamino ist z.B. Acetylamino oder Propionylamino.

Niederalkoxycarbonylamino ist z.B. Methoxycarbonylamino- oder Aethoxycarbonylamino.

Gegebenenfalls substituiertes Ureido ist z.B. Ureido oder 3-Niederalkyl- oder 3-Cycloalkyl-ureido, worin Cycloalkyl z.B. 5-7 Ringglieder hat, z.B. 3-Methylureido, 3-Aethylureido oder 3-Cyclohexylureido.

N-Niederalkylamino und N,N-Diniederalkylamino sind z.B. Methylamino, Aethylamino, Dimethylamino oder Diäthylamino.

Niederalkylenamino enthält vorzugsweise 5-7 Ringkohlenstoffatome und ist z.B. Pyrrolidino oder Piperidino.

Niederoxaalkylenamino ist in erster Linie Morpholino, während Niederazaalkylenamino insbesondere entsprechendes N-Niederalkyl-niederazaalkylenamino, z.B. 4-Methyl-1-piperazino, ist.

Unter den substituierten Niederalkylgruppen sind z.B. Hydroxyniederalkyl, Niederalkoxyniederalkyl, Halogenniederalkyl, Niederalkanoylaminoniederalkyl oder Niederalkoxycarbonylaminoniederalkyl zu nennen.

Hydroxyniederalkyl ist vorzugsweise Hydroxymethyl oder 1- und in erster Linie 2-Hydroxyäthyl.

Niederalkoxyniederalkyl ist vorzugsweise Niederalkoxymethyl oder 1- und in erster Linie 2-Niederalkoxyäthyl, z.B. Methoxymethyl, Aethoxymethyl, 2-Methoxy-äthyl oder 2-Aethoxy-äthyl.

Als Halogenniederalkylreste kommen insbesondere diejenigen in Betracht, die sich von den genannten Alkylresten ableiten und in denen das Halogenatom ein Bromatom oder insbesondere ein Chlor- oder Fluoratom ist, wie z.B. Chlormethyl, 2-Chloräthyl, Dichlormethyl und insbesondere Trifluormethyl.

Unter Niederalkoxycarbonylaminoniederalkylgruppen werden z.B. solche Reste verstanden, deren Niederalkylteile sich von den genannten Niederalkylgruppen ableiten. Solche Gruppen sind z.B. Methoxycarbonylaminomethyl, Aethoxycarbonylaminomethyl, 4-Methoxycarbonylamino-n-butyl, 2-Aethoxycarbonylaminoäthyl, 3-Aethoxycarbonylamino-n-propyl und besonders 2-Methoxycarbonylamino-äthyl und 3-Methoxycarbonylamino-n-propyl, Carbamoylmethyl oder 2-Carbamoyläthyl.

Niederalkanoylaminoniederalkyl ist insbesondere Niederalkanoylaminomethyl oder 1- und in erster Linie 2-Niederalkanoylamino-äthyl, z.B. Acetylaminomethyl, 2-Acetylamino-äthyl oder 2-Propionylamino-äthyl.

Die neuen Verbindungen besitzen wertvolle pharmakologische Eigenschaften. So zeigen sie eine lang anhaltende blutdrucksenkende Wirkung, wie sich im Tierversuch, z.B. bei i.v.-Gabe in Dosen von etwa 0,01-3,0 mg/kg an der narkotisierten Katze zeigen lässt.

Ferner besitzen die neuen Verbindungen eine noradrenolytische Wirkung, die sich in vitro, z.B. in Versuchen an isolierten vas deferens von Ratten in Konzentrationen von 0,001-0,1 µM zeigen lässt. Weiter bewirken die neuen Verbindungen Tachycardiehemmung, wie sich ebenfalls im Tierversuch zeigen lässt, z.B. in vitro Versuchen bei Konzentrationen von 1-2,7 µg/ml am isolierten Meeerschweinchenherz nach Langendorff ( Hemmung der Tachykardie durch Isoproterenol [5 x $10^{-9}$ µg/ml] bzw. Histamin [ 3 x $10^{-7}$ µg/ml]). Am isolierten Meerschweinchenvorhof senken diese Verbindungen die Spontanfrequenz und steigern die Kontraktionskraft, wirken somit negativ chromotrop und gleichzeitig positiv inotrop in Konzentrationen von 0,1 - 10 µg/ml. Ferner verlängern die neuen Verbindungen die funktionelle Refrakionszeit am isolierten Vorhof des Meerschweinchens.

Die neuen Verbindungen können demgemäss als antihypertensive Mittel oder als Mittel zur Behandlung von Herzinsuffizienz oder koronarer Herzkrankheit Verwendung finden. Ferner können sie als Ausgangs- oder Zwischenprodukte für die Herstellung anderer, insbesondere therapeutisch wirksamer Verbindungen dienen.

Besonders zu erwähnen sind die Verbindungen der Formel Ia

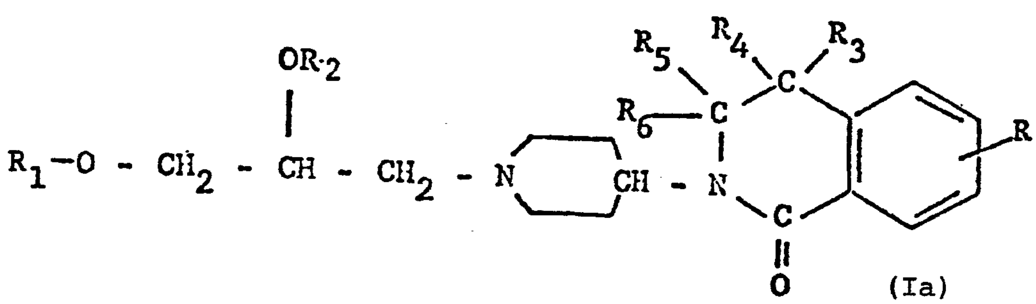

(Ia)

worin $R_1$ gegebenenfalls substituiertes, monocyclisches Heteroaryl oder Benzoheteroaryl mit 5 bis 6 Ringgliedern und 1 oder 2 Ringstickstoffatomen bedeutet, wobei Substituenten des heterocyclischen Arylrestes gegebenenfalls substituiertes Niederalkyl, z.B. Niederalkyl, Niederalkoxyniederalkyl, Niederalkanoylaminoniederalkyl oder Niederalkoxycarbonylaminoniederalkyl, oder gegebenenfalls veräthertes oder verestertes Hydroxy oder Mercapto, z.B. Niederalkoxy, Niederalkoxyniederalkoxy, Niederalkylthioniederalkoxy, Niederalkylthio oder Halogen, sein können $R_2$ einen niederen Alkanoylrest, wie insbesonders den Acetyl-, Propionyl- oder Pivaloylrest, oder vor allem ein Wasserstoffatom bedeutet, $R_3$ ein Wasserstoffatom oder die Hydroxylgruppe und $R_5$ ein Wasserstoffatom bedeutet oder $R_3$ und $R_5$ zusammen für eine zweite Bindung stehen und $R_4$ und $R_6$ je ein Wasserstoffatom bedeuten, oder $R_4$ zusammen mit $R_3$ für eine Oxogruppe steht und $R_5$ und $R_6$ je ein Wasserstoffatom bedeuten, oder $R_6$ zusammen mit $R_5$ für eine Oxogruppe steht und $R_3$ und $R_4$ je ein Wasserstoffatom bedeuten, und R einen Niederalkanoylaminorest, die Aminogruppe, die Nitrogruppe oder vor allem einen niederen Alkylrest, eine niedere Alkoxygruppe, ein Halogenatom, den Trifluormethylrest, die Hydroxylgruppe oder insbesondere ein Wasserstoffatom bedeutet, und ihren Salzen.

Die Erfindung betrifft insbesondere Verbindungen der Formel Ia, worin $R_1$ gegebenenfalls durch Niederalkyl, z.B. Methyl, Niederalkoxy, z.B. Methoxy, Niederalkylthio, z.B. Methylthio oder Aethylthio, und/oder mit Atomnummer bis zu 35, z.B. Chlor oder Brom, substituiertes monocyclisches Monoazaaryl oder Diazaaryl mit sechs Ringgliedern, wie Pyridyl, z.B. 2-, 3- oder 4-Pyridyl, Pyrimidinyl, z.B. 2- oder 4-Pyrimidinyl, oder Pyrazinyl, z.B. 2-Pyrazinyl darstellt, $R_2$ Niederalkanoyl, z.B. den Acetyl-, Propionyl- oder insbesondere Pivaloylrest oder vor allem ein Wasser-

stoffatom bedeutet, $R_3$ ein Wasserstoffatom oder die Hydroxylgruppe und $R_5$ ein Wasserstoffatom bedeutet oder $R_3$ und $R_5$ zusammen für eine zweite Bindung stehen und $R_4$ und $R_6$ je ein Wasserstoffatom bedeuten, oder $R_4$ zusammen mit $R_3$ für eine Oxogruppe steht und $R_5$ und $R_6$ je ein Wasserstoff bedeuten, oder $R_6$ zusammen mit $R_5$ für eine Oxogruppe steht und $R_3$ und $R_4$ je ein Wasserstoffatom bedeuten, wobei vor allem $R_4$ und $R_6$ je für Wasserstoff stehen und $R_3$ und $R_5$ entweder für eine zweite Bindung stehen oder je ein Wasserstoffatom bedeuten, und R einen niederen Alkylrest, z.B. Methyl, einen niederen Alkoxyrest, z.B. Methoxy, ein Halogenatom, z.B. Chlor, den Trifluoromethylrest oder insbesondere ein Wasserstoffatom bedeutet, und ihre Salze.

Die Erfindung betrifft insbesondere Verbindungen der Formel Ia, worin $R_1$ für gegebenenfalls durch Niederalkyl, z.B. durch Methyl, Niederalkoxy, z.B. Methoxy, Niederalkylthio, z.B. Methylthio oder Aethylthio, und/oder Halogen mit Atomnummer bis zu 35, z.B. Chlor oder Brom, substituiertes 2-Pyrazinyl, sowie für gegebenenfalls entsprechend substituiertes Pyridyl, z.B. 2- oder 3-Pyridyl steht, wobei Substituenten irgendeine Stellung, mindestens einer jedoch vorzugsweise die ortho-Stellung zum Verknüpfungsringkohlenstoffatom des Heteroarylrestes einnehmen können, und $R_2$, $R_3$, $R_4$, $R_5$, $R_6$ und R die im vorherigen Abschnitt angegebenen Bedeutungen haben oder ihre nicht-toxischen Säureadditionssalze.

Die Erfindung betrifft in erster Linie Verbindungen der Formel Ia, worin $R_1$ für 2-Pyrazinyl, ferner für Pyridyl, z.B. 2- oder 3-Pyridyl, steht, die in ortho-Stellung zum Verknüpfungskohlenstoffatom vorzugsweise durch Niederalkyl, z.B. Methyl, Niederalkoxy, z.B. Methoxy, Niederalkylthio, z.B. Methylthio oder Aethylthio, oder Halogen mit Atomnummer bis zu 35, z.B. Chlor oder Brom, substituiert sind, und gegebenenfalls weitere Substituenten dieser Art ent-

halten können, und $R_2$, $R_3$, $R_4$, $R_5$, $R_6$ und R Wasserstoff bedeuten, und Salze, insbesondere Säureadditionssalze, in erster Linie pharmazeutisch verwendbare, nicht-toxische Säureadditionssalze davon. Besonders zu nennen sind die in den Beispielen beschriebenen Verbindungen, insbesondere diejenigen der Formel I, die als $R_1$ einen vorzugsweise substituierten 2-Pyrazinylrest aufweisen, und $R_2$, $R_3$, $R_4$, $R_5$, $R_6$ und R Wasserstoff bedeuten, und ihre nicht-toxischen Säureadditionssalze.

Hervorzuheben sind auch Verbindungen der Formel Ib

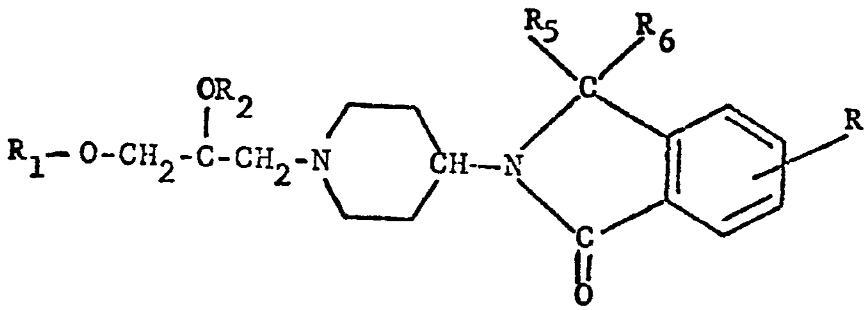

(Ib)

worin $R_1$ gegebenenfalls substituiertes, monocyclisches Heteroaryl oder Benzoheteroaryl mit 5 bis 6 Ringgliedern und 1 oder 2 Ringstickstoffatomen bedeutet, wobei Substituenten des heterocyclischen Arylrestes gegebenenfalls substituiertes Niederalkyl, z.B. Niederalkyl, Niederalkoxyniederalkyl, Niederalkanoylaminoniederalkyl oder Niederalkoxycarbonylaminoniederalkyl, oder gegebenenfalls veräthertes oder verestertes Hydroxy oder Mercapto, z.B. Niederalkoxy, Niederalkoxyniederalkoxy, Niederalkylthioniederalkoxy, Niederalkylthio oder Halogen sein können, und R je ein Wasserstoffatom, einen niederen Alkylrest, eine Hydroxygruppe, einen niederen Alkoxyrest, ein Halogenatom, eine Trifluormethylgruppe, die Nitrogruppe, eine Aminogruppe, eine Niederalkanoylaminogruppe, $R_2$ ein Wasserstoffatom oder eine niedere Alkanoylgruppe, insbesondere den Acetyl-, Propionyl- oder Pivaloylrest,

$R_5$ und $R_6$ je ein Wasserstoffatom, oder $R_5$ und $R_6$ zusammengenommen eine Oxogruppe bedeuten und ihre Salze. In den genannten Verbindungen befindet sich der Substituent R bevorzugt in meta-Stellung zur Carbonylgruppe und in para-Stellung zur Methylengruppe.

Die Erfindung betrifft insbesondere Verbindungen der Formel Ib, worin $R_1$ für gegebenenfalls durch Niederalkyl, z.B. durch Methyl, Niederalkoxy, z.B. Methoxy, Niederalkylthio, z.B. Methylthio oder Aethylthio, und/oder Halogen mit Atomnummer bis zu 35, z.B. Chlor oder Brom, substituiertes 2-Pyrazinyl, sowie für gegebenenfalls entsprechend substituiertes Pyridyl, z.B. 2- oder 3-Pyridyl steht, wobei Substituenten irgendeine Stellung, mindestens einer jedoch vorzugsweise die ortho-Stellung zum Verknüpfungsringkohlenstoffatom des Heteroarylrestes einnehmen können, R ein Wasserstoffatom, die Methyl-, Methoxy- oder Acetylaminogruppe, $R_2$ ein Wasserstoffatom, oder eine Niederalkanoyl-, z.B. die Acetyl-, Propionyl- oder die Pivaloylgruppe, $R_5$ und $R_6$ je ein Wasserstoffatom bedeuten, und ihre Salze.

Die Erfindung betrifft in erster Linie Verbindungen der Formel Ib, worin $R_1$ für 2-Pyrazinyl, ferner für Pyridyl, z.B. 2- oder 3-Pyridyl, steht, das in ortho-Stellung zum Verknüpfungskohlenstoffatom vorzugsweise durch Niederalkyl, z.B. Methyl, Niederalkoxy, z.B. Methoxy, Niederalkylthio, z.B. Methylthio oder Aethylthio, oder Halogen mit Atomnummer bis zu 35, z.B. Chlor oder Brom, substituiert ist, und gegebenenfalls weitere Substituenten dieser Art enthalten kann, und $R_2$ ein Wasserstoffatom, die Acetyl-, Propionyl- oder die Pivaloylgruppe und R, $R_5$ und $R_6$ ein Wasserstoffatom bedeuten, und ihre nicht-toxischen Säureadditionssalze.

Die Erfindung betrifft insbesondere Verbindungen der Formel Ib, worin $R_1$ gegebenenfalls durch Niederalkyl,

z.B. Methyl, Niederalkoxy, z.B. Methoxy, Niederalkylthio, z.B. Methylthio oder Aethylthio, und/oder Halogen mit Atomnummer bis zu 35, z.B. Chlor oder Brom, substituiertes monocyclisches Monoazaaryl oder Diazaaryl mit sechs Ringgliedern, wie Pyridyl, z.B. 2-, 3- oder 4-Pyridyl, Pyrimidinyl, z.B. 2- oder 4-Pyrimidinyl, oder Pyrazinyl, z.B. 2-Pyrazinyl darstellt, und R, $R_2$, $R_5$ und $R_6$ je ein Wasserstoffatom bedeuten, und ihre nicht-toxischen Säureadditionssalze.

Die neuen Verbindungen der Formel I werden nach an sich bekannten Methoden erhalten.

Man kann z.B. eine Verbindung der Formel

$$R_1 - O - Y_1 \qquad \text{(II)}$$

mit einer Verbindung der Formel

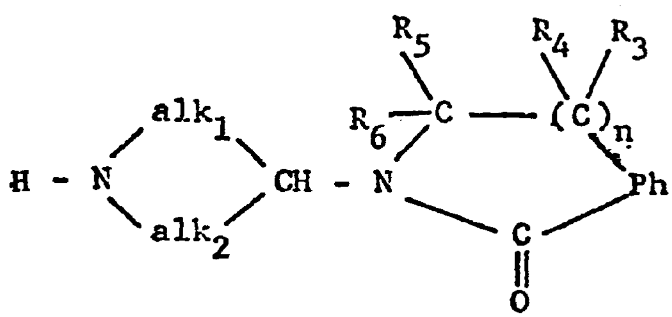

(III),

umsetzen, wobei $R_1$, $alk_1$, $alk_2$, Ph, n, $R_3$, $R_4$, $R_5$ und $R_6$ die angegebenen Bedeutungen haben, $Y_1$ für einen Rest der Formel

$$-CH_2-\underset{\displaystyle X}{\underset{|}{CH}}-CH_2-Z$$

steht, und X für die Gruppe $R_2$ steht, wobei $R_2$ die angegebene Bedeutung hat, und Z eine reaktionsfähige veresterte Hydroxylgruppe bedeutet, oder X und Z zusammen eine Epoxygruppe bilden.

Ferner kann man eine Verbindung der Formel

$$R_1 - OH \qquad (IIa),$$

worin $R_1$ obige Bedeutungen hat, mit einer Verbindung der Formel

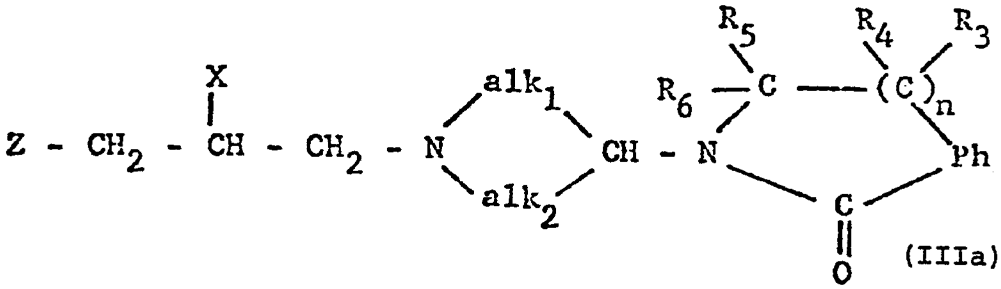

(IIIa),

worin X, Z, $alk_1$, $alk_2$, Ph, n, $R_3$, $R_4$, $R_5$ und $R_6$ obige Bedeutungen haben, umsetzen.

Diese Umsetzung wird in üblicher Weise durchgeführt. Falls reaktionsfähige Ester als Ausgangsmaterial verwendet werden, kann die Verbindung der Formel IIa vorzugsweise in Form ihres Metall-Phenolats, wie Alkali-Phenolats, beispielsweise Natrium-Phenolats, verwendet werden, oder man arbeitet in Gegenwart eines säurebindenden Mittels, insbesondere eines Kondensationsmittels, welches mit der Verbindung der Formel IIa ein Salz bilden kann, wie ein Alkalialkoholat.

Ferner kann man die neuen Verbindungen, in denen $R_3$ Hydroxy ist, dadurch herstellen, dass man in einer Verbindung der Formel

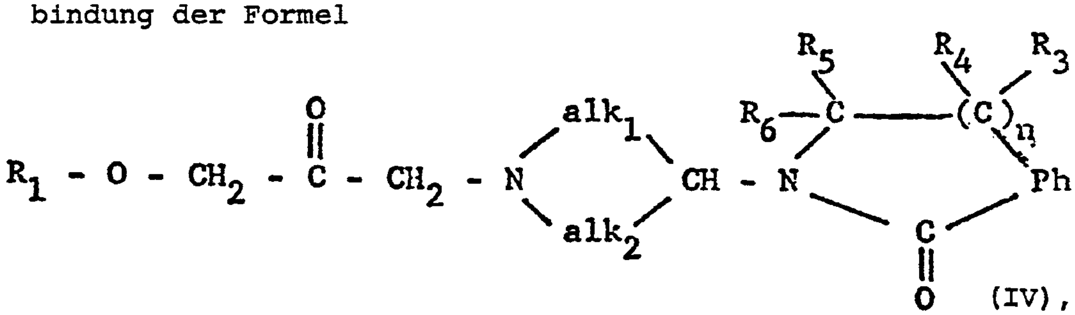

(IV),

worin $R_1$, $alk_1$, $alk_2$, Ph, n, $R_3$,$R_4$,$R_5$ und $R_6$ obige Bedeutungen haben, die Oxogruppe der Propylkette zu einer Hydroxylgruppe reduziert.

Diese Reduktion wird in üblicher Weise, insbesondere unter Verwendung eines Dileichtmetallhydrids, wie Natriumborhydrid durchgeführt. Die Reduktion kann aber auch mit nascierendem Wasserstoff durchgeführt werden. Nascierender Wasserstoff kann dabei durch Einwirkung von Metall oder Metall-Legierungen auf Wasserstoff liefernde Mittel, wie Carbonsäure, Alkohole oder Wasser erhalten werden, wobei insbesondere Zink oder Zinklegierungen zusammen mit Essigsäure oder Alkalimetall und Alkohol, wie Natrium und Aethanol, in Betracht kommen.

Ausserdem kann die Reduktion durch katalytische Hydrierung durchgeführt werden, wie mit Wasserstoff in Gegenwart eines Hydrierungskatalysators, beispielsweise Schwermetalle wie Palladium, Platin oder Raney-Nickel. Bei der Reduktion muss darauf geachtet werden, dass andere reduzierbare Gruppen nicht angegriffen werden.

Ferner kann man die neuen Verbindungen erhalten, wenn man in einer Verbindung der Formel

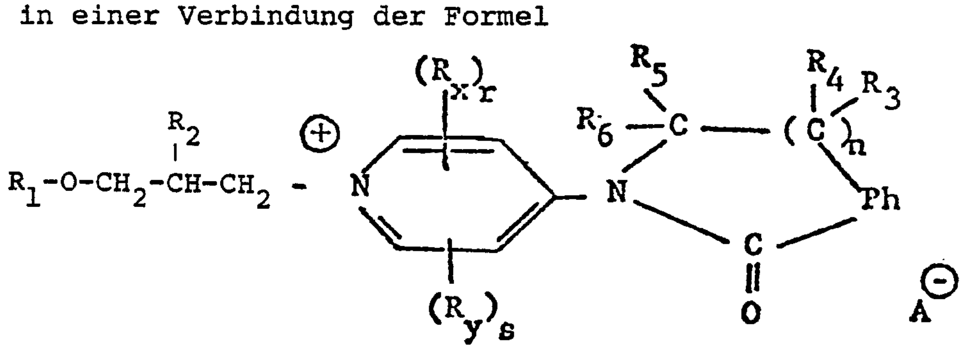

(V),

worin $R_1$, $R_2$, $R_3$, $R_4$, $R_5$, $R_6$, Ph und n obige Bedeutungen haben, $R_x$ und $R_y$ unabhängig voneinander niedere Alkylreste oder Wasserstoffatome bedeuten und r und s für 1 oder 2 stehen, und $A^{\ominus}$ ein Anion bedeutet, den Pyridiniumring zum Piperidinring reduziert.

Die Reduktion kann in üblicher Weise durchgeführt werden, vorzugsweise durch katalytische Hydrierung, wie mit Wasserstoff in Gegenwart eines Hydrierungskatalysators, beispielsweise Schwermetalle, wie Palladium, Platin oder Raney-Nickel, oder mit nascierendem Wasserstoff, wie beispielsweise Natrium und Alkohol, wie Niederalkanol, z.B. Aethanol. Bei der Reduktion muss darauf geachtet werden, dass andere reduzierbare Gruppen nicht angegriffen werden.

Ferner kann man die neuen Verbindungen erhalten, indem man eine Verbindung der Formel

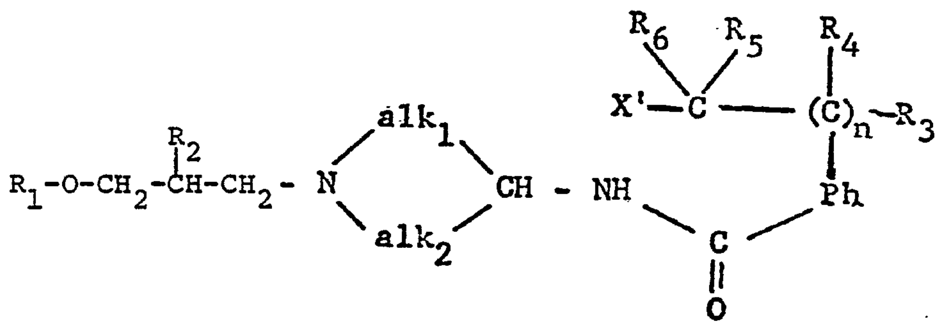

(VI),

worin $R_1$, $R_2$, $alk_1$, $alk_2$, Ph, n, $R_3$, $R_4$, $R_5$ und $R_6$ die angegebenen Bedeutungen haben und X' eine reaktionsfähige veresterte Hydroxylgruppe bedeutet, intramolekular kondensiert.

Eine reaktionsfähige veresterte Hydroxylgruppe ist insbesondere eine der oben genannten.

Die Cyclisierung (intramolekulare Kondensation) kann in üblicher Weise erfolgen, vorzugsweise in Gegenwart eines Lösungsmittels, wie eines inerten, polaren Lösungsmittels wie eines Alkohols, z.B. Aethanol oder Isopropanol, oder von Dimethylformamid und vorteilhaft in Anwesenheit eines Kondensationsmittels, besonders eines basischen Kondensationsmittels. Vorzugsweise arbeitet man in Gegenwart eines Alkali- oder Erdalkali-hydroxyds, -carbonats oder -bicarbonats, z.B. Natriumhydroxid, Kaliumhydroxyd, Calciumhydroxyd, Natriumcarbonat, Kaliumcarbonat, Calciumcarbonat, Natriumbicarbonat oder Kaliumbicarbonat, oder eines Alkaliacetats, wie Natriumacetat, oder eines Alkalialkoholats, wie Natriummethylat, oder organischen tertiären Stickstoffbasen, wie Trialkylamine, z.B. Trimethylamin oder Triäthylamin, oder Pyridin.

Die neuen Verbindungen, in denen $R_3$ und $R_5$ zusammen für eine zweite Bindung stehen, wenn n = 1 ist, können auch erhalten werden, wenn man eine Verbindung der Formel

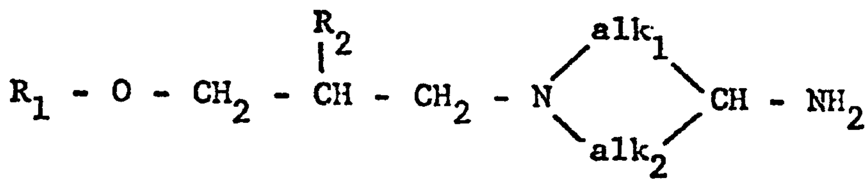

(VII),

mit einer Verbindung der Formel

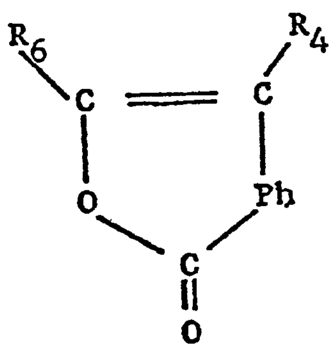

(VIII),

worin $R_1$, $R_2$, $R_4$, $R_6$, $alk_1$, $alk_2$ und Ph die angegebenen Bedeutungen haben, umsetzt.

Die Umsetzung kann in an sich bekannter Weise erfolgen. Vorteilhaft arbeitet man in Gegenwart einer organischen Base, wie einem tertiären Amin, vor allem Pyridin, wobei diese Base auch gleichzeitig als Lösungsmittel dienen kann. Man kann aber auch in Gegenwart weiterer Lösungsmittel arbeiten.

Ferner kann man die neuen Verbindungen erhalten, wenn man eine Verbindung der Formel

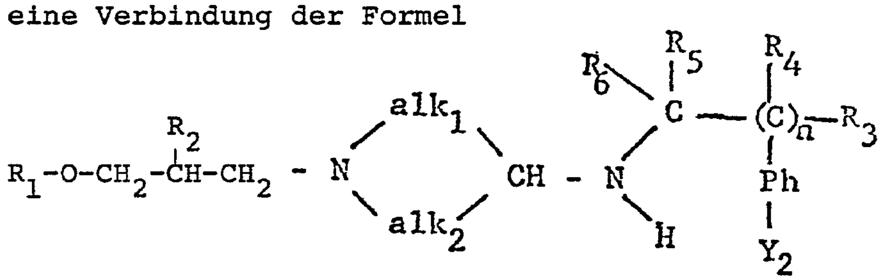

(IX),

worin $R_1$, $R_2$, $alk_1$, $alk_2$, $R_3$, $R_4$, $R_5$, $R_6$, n und Ph die angegebenen Bedeutungen haben und $Y_2$ eine freie oder vorzugsweise eine Oxogruppe enthaltende funktionell abgewandelte Carboxylgruppe bedeutet, intramolekular kondensiert.

Eine eine Oxogruppe enthaltende funktionell abgewandelte Carboxylgruppe ist beispielsweise eine veresterte Carboxylgruppe wie insbesondere mit einem niederen Alkanol oder Aralkanol, wie Methanol, Phenol, p-Nitrophenol, oder Benzylalkohol veresterte Carboxylgruppe oder eine aktivierte veresterte Carboxylgruppe, wie eine mit Cyanmethanol veresterte Carboxylgruppe, oder eine Säurehalogenid-, wie insbesondere Säurechlorid-gruppierung oder eine Säureazid-, Säureamid- oder Säureanhydridgruppierung. Als Säureanhydridgruppierung kommen insbesondere solche von gemischten Anhydriden, insbesondere von gemischten Anhydriden mit Kohlensäuremonoalkylestern, wie Kohlensäuremonoäthyl- oder -isobutylester in Betracht.

Die Umsetzung kann in üblicher Weise erfolgen. Vorzugsweise arbeitet man bei erhöhter Temperatur. Die Umsetzung wird vorteilhaft in einem Lösungsmittel, wie einem inerten Lösungsmittel, z.B. einen Kohlenwasserstoff, wie Benzol oder Toluol oder in einem hochsiedenden inerten Lösungsmittel wie z.B. Diphenyläther durchgeführt.

Ferner kann man die neuen Verbindungen, in denen $R_2$ für Wasserstoff steht, erhalten, wenn man in einer Verbindung der Formel

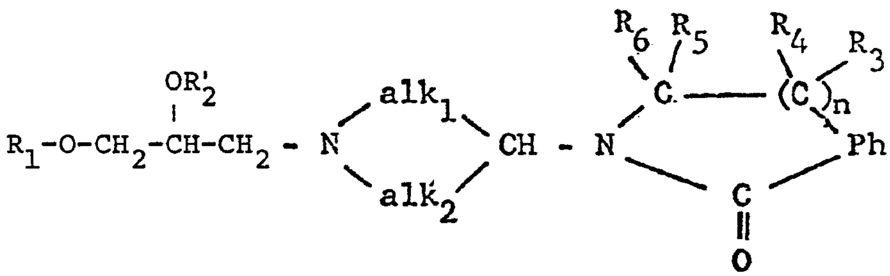

(X),

worin $R_1$, $alk_1$, $alk_2$, Ph, n, $R_3$, $R_4$, $R_5$ und $R_6$ die angegebenen Bedeutungen haben und $R_2'$ einen durch Hydrogenolyse abspaltbaren Rest bedeutet, $R_2'$ durch Hydrogenolyse abspaltet.

Ein durch Hydrogenolyse abspaltbarer Rest ist vor allem ein α-Aralkylrest, wie der Benzylrest oder ein α-Aralkoxycarbonylrest, wie der Carbobenzoxyrest. Die Hydrogenolyse kann in üblicher Weise erfolgen, vorzugsweise mittels Wasserstoff in Gegenwart eines Hydrierungskatalysators, wie eines Nickel-, Palladium-, Platin- oder Rutheniumkatalysators.

Ferner kann man die neuen Verbindungen der Formel I, worin $R_6$ zusammen mit $R_5$ eine Oxogruppe bildet, erhalten, wenn man eine Verbindung der Formel

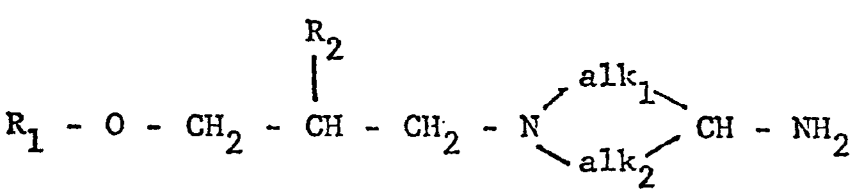

(VII),

worin $R_1$, $R_2$ $alk_1$ und $alk_2$ obige Bedeutungen haben, mit einer Verbindung der Formel

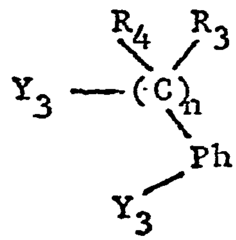

(XI),

worin $R_3$, $R_4$, n und Ph obige Bedeutungen haben und die Substituenten $Y_3$ unabhänig voneinander für eine freie, oder vorzugsweise eine eine Oxogruppe enthaltende funktionell abgewandelte Carboxylgruppe oder zusammen für die Gruppierung

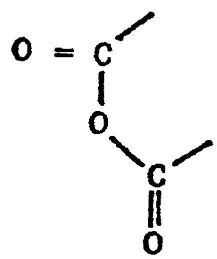

stehen, umsetzt.

Eine eine Oxogruppe enthaltende funktionell abgewandelte Carboxylgruppe ist beispielsweise eine veresterte Carboxylgruppe wie insbesondere mit einem niederen Alkanol oder Aralkanol, wie Methanol, Phenol, p-Nitrophenol, oder Benzylalkohol veresterte Carboxylgruppe oder eine aktivierte veresterte Carboxylgruppe, wie eine mit Cyanmethanol veresterte Carboxylgruppe, oder eine Säurehalogenid-,wie insbesondere Säurechlorid-gruppierung oder eine Säureazid-, Säureamid- oder Säureanhydridgruppierung. Als Säureanhydridgruppierung kommen insbesondere solche von gemischten Anhydriden, insbesondere von gemischten Anhydriden mit Kohlensäuremonoalkylestern, wie Kohlensäuremonoäthyl- oder -isobutylester in Betracht.

Die Umsetzung kann in üblicher Weise erfolgen. Vorzugsweise arbeitet man bei erhöhter Temperatur. Die Umsetzung wird vorteilhaft in einem Lösungsmittel, wie einem inerten

Lösungsmittel, z.B. einen Kohlenwasserstoff, wie Benzol oder Toluol oder in einem hochsiedenden inerten Lösungsmittel wie z.B. Diphenyläther durchgeführt.

In erhaltenen Verbindungen kann man im Rahmen der Endstoffe Substituenten abspalten, einführen oder umwandeln.

So kann man in Verbindungen der Formel I mit ungesättigten Substituenten, z.B. Niederalkenyl, Niederalkenyloxy oder Niederalkinyloxy, diese durch geeignete Reduktionsmethoden zu entsprechenden gesättigten Verbindungen oder, im Fall von Substituenten mit einer Dreifachbindung, zu Verbindungen mit einer Doppelbindung reduzieren. Dabei verwendet man als Reduktionsmittel vorzugsweise katalytisch aktivierten Wasserstoff, im Fall einer Dreifachbindung auch ein chemisches Reduktionsmittel, wie Natrium in Gegenwart von flüssigem Ammoniak.

Man kann auch in einer erhaltenen Verbindung der Formel I, die als Substituenten eines aromatischen Restes Halogen, wie Brom oder Jod, enthält, dieses z.B. durch Behandeln mit Trifluormethyljodid in Gegenwart von Kupferpulver und eines geeigneten aprotischen Lösungsmittels, wie Pyridin, Dimethylformamid oder Acetonitril, durch Trifluormethyl ersetzen.

In einer erhaltenen Verbindung der Formel I kann man eine α-Phenylniederalkylgruppe, z.B. in Benzyloxy, durch Behandeln der entsprechenden Verbindung mit katalytisch aktiviertem Wasserstoff abspalten und durch Wasserstoff, z.B. eine Benzyloxygruppe durch Hydroxy, ersetzen.

Ferner kann man in einer Verbindung der Formel I, die Hydroxy oder Mercapto in der Form einer primären Carbinol- oder einer phenolischen Hydroxylgruppe als Substituenten enthält, dieses, gegebenenfalls in Salz-, z.B. Alkalimetallsalzform, durch Behandeln mit einem reaktionsfähigen Ester eines Alkohols, wie einem gegebenenfalls substituierten Niederalkylhalogenid, in veräthertes Hydroxy oder Mercapto, z.B. Niederalkoxy bzw. Niederalkylthio, umwandlen. Zudem kann man Hydroxy in einem Hydroxyniederalkyl- oder Hydroxyniederalkoxy-Substituenten, üblicherweise in Form einer reaktionsfähigen veresterten Hydroxygruppe, wie Halogen, z.B. Chlor, mit einem Alkohol, z.B. Niederalkanol, oder einem Mercaptan, z.B. Niederalkylmercaptan, vorzugsweise in Gegenwart eines basischen Mittels, das z.B. einen Alkohol oder ein Mercaptan in eine Metallverbindung überzuführen vermag, umsetzen und so zu Verbindungen der Formel I gelangen, die entsprechend veräthertes Hydroxy- oder Mercapto-niederalkyl bzw. -niederalkoxy aufweisen. Ferner kann man in einer erhaltenen Verbindung eine reaktionsfähige veresterte Hydroxygruppe, wie Halogen, z.B. Chlor, insbesondere in α-Stellung zu einem Ringstickstoffatom in einem Rest $R_1$, z.B. durch Behandeln mit einer Alkoholat- oder Thio-alkoholatverbindung, wie einem Alkalimetall-, z.B. Natrium- oder Kalium-niederalkanolat oder -thioniederalkanolat, in eine verätherte oder veresterte Hydroxy- oder Mercaptogruppe, z.B. in Niederalkoxy oder Niederalkylthio, umwandeln.

In einer Verbindung der Formel I kann man eine Propargyloxygruppe, z.B. durch Hydratisierung in saurem Medium und in Gegenwart eines Quecksilber-II-salzes, z.B. durch Behandeln mit einer wässrigen Mineralsäure, z.B. verdünnter Salz- oder Schwefelsäure, in Gegenwart von Quecksilber-II-chlorid, in die Acetonyloxygruppe umwandeln.

Man kann auch in einer Verbindung der Formel I, die primäres Amino als Substituenten enthält, dieses substituieren; so kann man Amino, z.B. durch Behandeln der Aminoverbindung mit einem geeigneten Säurederivat, wie einem gegebenenfalls gemischten Anhydrid, z.B. einem

entsprechenden Chlorid, falls notwendig in Gegenwart eines basischen Mittels, acylieren.

Weiterhin kann man in Verbindungen, der Formel I, worin Ph einen eine Aminogruppe enthaltenden o-Phenylenrest bedeutet, die genannte Aminogruppe acylieren, wie z.B. durch Umsetzung mit einem Acylierungsmittel.

Als Acylierungsmittel kommen Carbonsäuren, beispielsweise aliphatische, araliphatische oder cycloaliphatische Carbonsäuren, vorzugsweise in Form ihrer funktionellen Derivate, wie Halogenide, insbesondere Chloride, oder Anhydride, z.B. reine oder gemischte Anhydride, oder innere Anhydride, wie Ketene, in Betracht.

Ferner kann man in Verbindungen der Formel I, die Hydroxylgruppen enthalten, diese acylieren (verestern). Die Acylierung erfolgt in üblicher Weise, z.B. durch Umsetzung mit Carbonsäuren, vorteilhaft in Form ihrer reaktionsfähigen funktionellen Derivate, wie Säurehalogenide, z.B. Chloride, Ester, insbesondere Ester mit niederen Alkanolen, wie Methanol und Aethanol, oder aktivierte Ester wie Cyanmethylester, oder reine oder gemischte Anhydride, z.B. gemischte Anhydride mit Kohlensäure-monoalkylestern wie Kohlensäuremonoäthyl- und -isobutylester.

In Verbindungen der Formel I, die eine acylierte Hydroxy- oder Aminogruppe enthalten, kann man diese in üblicher Weise zur freien Hydroxyl- bzw. Aminogruppe spalten, insbesondere hydrolytisch, je nach Zweckmässigkeit sauer oder basisch katalysiert, z.B. mit anorganischen Säuren oder Alkalilaugen (Basen), z.B. mit Salzsäure oder mit Natronlauge. Sollte eine solche Spaltung bereits im Verlauf einer der obigen Herstellungsmethoden eintreten, so kann eine erhaltene freie Hydroxyl- oder Aminogruppe gegebenenfalls wie oben beschrieben acyliert werden.

Ferner kann in Verbindungen der Formel I, welche Substituenten mit einer C-C-Doppel- oder -Dreifach-Bindung enthalten, die C-C-Doppel bzw. -Dreifach-Bindung durch katalytische Hydrierung, wie durch Wasserstoff in Gegenwart eines Hydrierungskatalysators, beispielsweise Nickel, Platin oder Palladium, wie Raney-Nickel, Platinschwarz oder Palladium auf Aktivkohle, in eine C-C-Einfachbindung überführt werden. Dabei muss darauf geachtet werden, dass andere reduzierbare Gruppen, nicht angegriffen werden.

In Verbindungen der Formel I, welche Substituenten mit einer C-C-Dreifachbindung enthalten, kann diese ferner lediglich zu einer C-C-Doppelbindung und wenn erwünscht stereospezifisch zu einer C-C-cis- oder C-C-trans-Doppelbindung reduziert werden. Die Reduktion einer C-C-Dreifachbindung zu einer C-C-Doppelbindung kann beispielsweise durch Hydrierung mit 1 Mol Wasserstoff in Gegenwart eines weniger aktiven Hydrierungskatalysators, wie Eisen oder Palladium, beispielsweise Raney-Eisen oder Palladium auf Bariumsulfat, insbesondere bei erhöhter Temperatur, erfolgen. Die Reduktion zu einer C-C-cis-Doppelbindung kann beispielsweise mittels 1 Mol Wasserstoff in Gegenwart eines desaktivierten Katalysators, wie Palladium auf Tierkohle in Gegenwart von Chinolin, Palladium auf Calziumcarbonat in Gegenwart von Bleisalzen, oder auch Raney-Nickel, erfolgen. Die Reduktion zu einer C-C-trans-Doppelbindung kann beispielsweise mittels Natrium in flüssigem Ammoniak erfolgen, wobei insbesondere mit Rücksicht auf eine Harnstoffgruppe kurze Reaktioszeiten und kein Ueberschuss an Reduktionsmittel angewendet werden, und wobei gegebenenfalls ein Ammoniumhalogenid, wie Ammoniumchlorid, als Katalysator zugegeben wird.

In erhaltenen Verbindungen der Formel I, die an einem aromatischen Kern Nitrogruppen aufweisen, kann man diese zu Aminogruppen reduzieren.

Die Reduktion kann in üblicher Weise erfolgen, z.B. durch nascierenden Wasserstoff (z.B. mit Eisen und Salzsäure oder mit Aluminiumamalgam) oder mit katalytisch erregtem Wasserstoff, wie Wasserstoff in Gegenwart von Platin-, Nickel- oder Palladiumkatalysatoren.

Ferner kann man Verbindungen der Formel I, in denen $R_3$ und $R_4$ zusammen für eine Oxogruppe stehen, zu Verbindungen reduzieren, worin $R_3$ für Hydroxyl und $R_4$ für Wasserstoff steht.

Die Reduktion der Oxogruppe erfolgt in üblicher Weise, z.B. durch metallische Reduktion, wie durch Behandeln mit Natrium in Alkohol, oder mit komplexen Metallhydriden,wie Natriumborhydrid, oder durch katalytisch erregten Wasserstoff, z.B. Wasserstoff in Gegenwart eines Platin-, Palladium-, Nickel- oder Kupferkatalysators, wie Platinoxyd, Palladiumkohle, Raney-Nickel oder Kupferchromit. Die Umsetzung erfolgt vorzugsweise in Anwesenheit von Verdünnungs- und/oder Lösungsmitteln, bei tiefer, gewöhnlicher oder erhöhter Temperatur, im offenen oder im geschlossenen Gefäss unter Druck.

Die Reduktion der Oxogruppe kann auch nach der Meerwein-Ponndorf-Verley-Methode erfolgen. So kann man beispielsweise die Oxoverbindung in üblicher Weise mit einem niederen Alkanol wie Isopropanol in Gegenwart eines entsprechenden Alkanolats, wie Aluminiumisopropylat, behandeln.

Ferner kann man in Verbindungen der Formel I, worin $R_3$ für Hydroxyl steht die Hydroxylgruppe abspalten. Man erhält so Verbindungen, in denen $R_3$ und $R_5$ für eine zweite Bindung stehen.

Die Abspaltung kann in üblicher Weise erfolgen, z.B. durch Behandlung mit starken Säuren, wie Schwefelsäure, p-Toluolsulfonsäure, konzentrierter Salzsäure, Oxalsäure oder andere wasserabspaltenden Mitteln, wie Phosphorpentoxyd, Zinkchlorid oder Bortrioxid. Gegebenenfalls entfernt man das Wasser mittels eines Wasserabscheiders. Beispielsweise kann man die Reaktion in einem siedendem Kohlenwasserstoff, wie Benzol oder Toluol durchführen.

Ferner kann man Verbindungen der Formel I, worin $R_3$ für Hydroxyl steht, die Hydroxylgruppe durch Wasserstoff ersetzen. Dies kann beispielsweise durch katalytische Hydrierung geschehen.

Ferner kann man in Verbindungen der Formel I, worin $R_3$ und $R_5$ für eine zweite Bindung stehen, zu Verbindungen hydrieren, in denen $R_3$ und $R_5$ für Wasserstoffatome stehen. Dies kann insbesondere durch katalytische Hydrierung erfolgen.

Die katalytische Hydrierung kann in üblicher Weise durchgeführt werden, insbesondere mittels Wasserstoff in Gegenwart eines Hydrierungskatalysators, wie eines Palladium-, Platin- oder Nickelkatalysators.

Die genannten Reaktionen können gegebenenfalls gleichzeitig oder nacheinander und in beliebiger Reihenfolge durchgeführt werden.

Die genannten Reaktionen können in üblicher Weise in An- oder Abwesenheit von Lösungs- oder Verdünnungsmitteln, sauren oder basischen Kondensationsmitteln und/oder Katalysatoren bei erniedrigter, gewöhnlicher oder erhöhter Temperatur, gegebenenfalls im gelschlossenen Gefäss unter erhöhtem Druck und/oder unter einer Inertgasatmosphäre durchgeführt werden.

Je nach den Verfahrensbedingungen und Ausgangsstoffen erhält man die Endstoffe in freier Form oder in der ebenfalls in der Erfindung inbegriffenen Form ihrer Säureadditionssalze. So können beispielsweise basische, neutrale oder gemischte Salze, gegebenenfalls auch Hemi-, Mono-, Sesqui- oder Polyhydrate davon, erhalten werden. Die Säureadditionssalze der neuen Verbindungen können in an sich bekannter Weise in die freie Verbindung übergeführt werden, z.B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, z.B. Chlorwasserstoffsäure, Schwefelsäuren, z.B. Schwefelsäure, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Ascorbin-, Malein-, Hydroxymalein- oder Benztrauben-, Fumar-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Tryptophan, Lysin oder Arginin.

Diese oder andere Salze der neuen Verbindungen, wie z.B. die Pikrate, können auch zur Reinigung der erhaltenen freien Basen dienen, indem man die freien Basen in Salze überführt, diese abtrennt und aus den Salzen wiederum die Basen frei macht. Infolge der engen Beziehungen zwischen den neuen Verbindungen in freier Form und in Form ihrer Salze sind im Vorausgegangenen und nachfolgend unter den

freien Verbindungen sinn- und zweckmässig gegebenenfalls auch die entsprechenden Salze zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangstoff unter den Reaktionsbedingungen bildet, oder bei denen eine Reaktionskomponente gegebenenfalls in Form eines optischen Antipoden und/oder eines Salzes vorliegt.

So kann man z.B. die neuen Piperidine erhalten, wenn man eine Verbindung der Formel

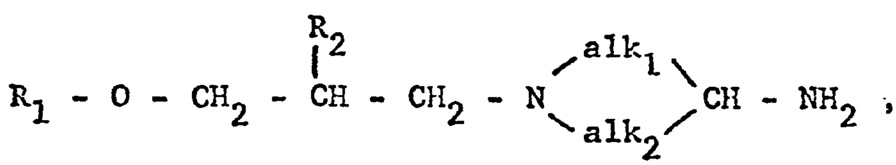

worin $R_1$, $R_2$, $alk_1$ und $alk_2$ die angegebenen Bedeutungen haben, mit einer Verbindung der Formel

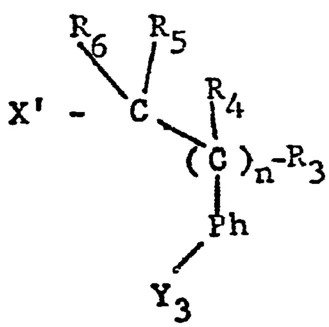

umsetzt, worin n, $R_3$, $R_4$, $R_5$ und $R_6$ die angegebenen Bedeutungen haben und X' und $Y_3$ die bei den Formeln VI bzw. IX angegebenen Bedeutungen haben. Dabei entsteht intermediär eine Verbindung der Formel VI, die dann erfindungsgemäss zur Verbindung der Formel I weiterreagiert. Die Umsetzung kann in üblicher Weise durchgeführt werden, z.B. wie oben für die intramolekularen Kondensationen beschrieben.

Die neuen Verbindungen können je nach der Wahl der Ausgangsstoffe und Arbeitsweisen als optische Antipoden oder Racemate oder, sofern sie mindestens zwei asymmetrische Kohlenstoffatome enthalten, auch als Racematgemische und/oder als reine geometrische Isomere oder als Gemische derselben (Isomerengemische) vorliegen.

Erhaltene Isomerengemische können aufgrund der physikalisch-chemischen Unterschiede der Bestandteile in bekannter Weise in die beiden reinen geometrischen Isomeren aufgetrennt werden, beispielsweise durch Chromatographie in einer geeigneten stationären Phase, wie mit einer komplexbildenden Schwermetallverbindung, z.B. mit einer Silberverbindung, vorbehandeltem Kieselgel oder Aluminiumoxid, oder durch Bildung einer Schwermetalladditionsverbindung, z.B. des Silbernitrat-Komplexes, Trennung derselben in die Additionsverbindungen der reinen Isomeren, z.B. durch fraktionierte Kristallisation, und anschliessende Freisetzung der reinen Isomeren.

Erhaltene reine Isomere, z.B. trans-Isomere, können in üblicher Weise, z.B. photochemisch, beispielsweise durch Bestrahlen mit Licht geeigneter Wellenlänge, vorteilhaft in einem geeigneten Lösungsmittel, wie einem aliphatischen Kohlenwasserstoff, oder in Gegenwart eines geeigneten Katalysators, in die jeweils entgegengesetzt konfiguierten Isomeren, z.B. in die cis-Isomeren, umgewandelt werden.

Racematgemische können aufgrund der physikalisch-chemischen Unterschiede der Bestandteile in bekannter Weise in die beiden stereoisomeren (diastereomeren) reinen Racemate aufgetrennt werden, beispielsweise durch Chromatographie

und/oder fraktionierte Kristallisation.

Erhaltene Racemate lassen sich nach bekannten Methoden, beispielsweise durch Umkristallisation aus einem optisch aktiven Lösungsmittel, mit Hilfe von Mikroorganismen oder durch Umsetzen mit einer mit der racemischen Verbindung Salze bildenden optisch aktiven Säure und Trennung der auf diese Weise erhaltenen Salze, z.B. aufgrund ihrer verschiedenen Löslichkeiten, in die Diastereomeren, aus denen die Antipoden durch Einwirkung geeigneter Mittel freigesetzt werden können, zerlegen. Besonders gebräuchliche optisch aktive Säuren sind z.B. die D- und L-Formen von Weinsäure, Di-o-Toluolweinsäure, Aepfelsäure, Mandelsäure, Camphersulfonsäure oder Chinasäure. Vorteilhaft isoliert man den wirksameren L-Antipoden.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden erhalten werden.

Die als bevorzugte Ausgangsstoffe verwendeten Verbindungen der Formel

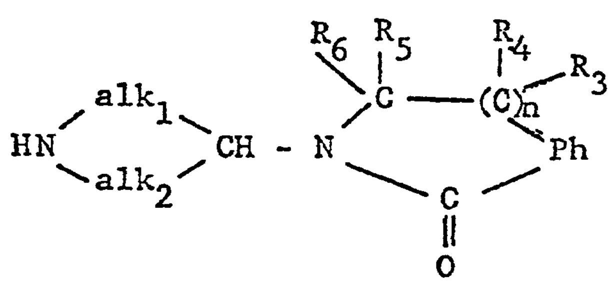

(IIIa)

kann man beispielsweise erhalten, wenn man eine Verbindung der Formel

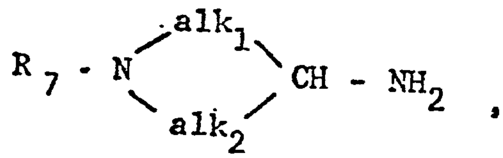

worin $alk_1$ und $alk_2$ die angegebenen Bedeutungen haben und $R_7$ einen α-Aralkylrest, wie einen Benzylrest, bedeutet, mit einer Verbindung der Formel

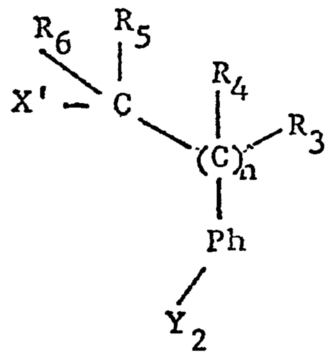

umsetzt, worin n, $R_3$, $R_4$, $R_5$ und $R_6$ die angegebenen Bedeutungen haben und X' und $Y_2$ die bei den Formeln VI bzw. IX angegebenen Bedeutungen haben, und in der erhaltenen Verbindung der Formel

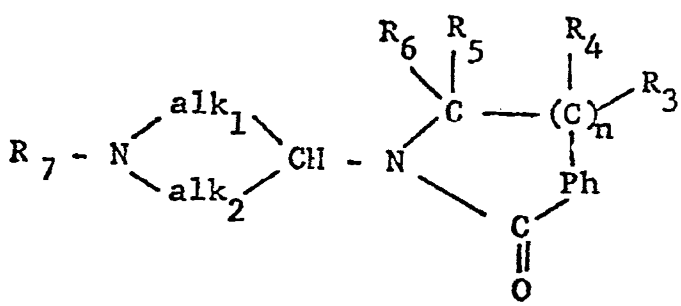

den α-Aralkylrest $R_7$ durch Wasserstoff ersetzt, z.B. durch katalytische Hydrierung wie oben beschrieben.

Die neuen Verbindungen können als Heilmittel, z.B. in Form pharmazeutischer Präparate, Verwendung finden, welche sie oder ihre Salze in Mischung mit einem z.B. für die enterale, z.B. orale, oder parenterale Applikation geeigneten pharmazeutischen, organischen oder anorganischen, festen oder flüssigen Trägermaterial enthalten. Für die Bildung desselben kommen solche Stoffe in Frage, die mit den neuen Verbindungen nicht reagieren, wie z.B. Wasser, Gelatine, Milchzucker, Stärke, Magnesiumstearat, Talk, Pflanzliche Oele, Benzylalkohole, Gummi, Polyalkylenglykole, Vaseline, Cholesterin oder andere bekannte Arzneimittelträger. Die pharmazeutischen Präparate können z.B. als Tabletten, Dragées, Kapseln, Suppositorien, Salben, Crèmen oder in flüssiger Form als Lösungen (z.B. als Elixier oder Sirup), Suspensionen oder Emulsionen vorliegen. Gegebenenfalls sind sie sterilisiert und bzw. oder enthalten Hilfsstoffe, wie Konservierungs-, Stabilisierungs-, Netz oder Emulgiermittel, Salze zur Veränderung des osmotischen Druckes oder Puffer. Sie können auch noch andere therapeutisch wertvolle Stoffe enthalten. Die Präparate welche auch in der Veterinärmedizin Verwendung finden können, werden nach üblichen Methoden gewonnen.

Die Dosierung der neuen Verbindungen hängt von der Art der zu behandelnden Zustände und von den individuellen Bedürfnissen ab. Beispielsweise kann man die neuen Verbindungen einem Warmblütler von ungefähr 75 kg Körpergewicht in einer täglichen Dosis von etwa 5-100 mg, insbesondere etwa 5 bis 60 mg verabreichen.

Die neuen Verbindungen können auch vorteilhafterweise in Kombination mit anderen Antihypertensiva und/oder Diuretica in pharmazeutischen Präparaten verwendet werden.

Als antihypertensiv wirksame Verbindungen kommen insbesondere solche vom Typ der α-Amino-β-hydroxyphenylpropionsäure, der β-Amino-β-alkoxyphenyl-propionsäure, und besonders der Hydrazinopyridazine und der Sympathicolytica in Betracht.

Geeignete Diuretica sind Stoffe, die sowohl durch renale als auch durch extrarenale Wirkung auf die Gewebe die Diurese erhöhen. Dabei kommen Substanzen mit hemmender Wirkung auf die Rückresorption im Tubulus, wie z.B. besonders Saluretica sowie Aethacrinsäure und deren Analoge in Betracht.

Insbesondere geeignet sind Benzothiadiazin-Derivate, wie Thiazide und Hydrothiazide, Benzolsulfonamide, Phenoxyessigsäuren, Benzofuran-2-carbonsäuren und Benzofuran-2,3-dihydro-2-carbonsäuren.

Die folgenden Beispiele erläutern die Erfindung ohne sie jedoch einzuschränken. Die Temperaturen sind in Celsiusgraden angegeben.

Beispiel 1

24.2 g (0.13 Mol) 2-Chlor-3-(2,3-epoxypropoxy)pyrazin und 23 g (0.1 Mol) 1-(4-Piperidinyl)-3,4-dihydro-1(2H)-isochinolon in 250 ml Isopropanol werden 24 Stunden bei ca. 20°C gerührt. Die ausgefallenen Kristalle werden abgenutscht, mit Aether gewaschen und man erhält 36,6 g 2-{1-[3-(3-Chlor-2-pyrazinyloxy)-2-hydroxy-1-propyl]-4-piperidyl}-3,4-dihydro-1(2H)-isochinolon, Smp. 115-116°.

Das mit alkoholischer Salzsäure hergestellte Hydrochlorid kristallisiert aus Alkohol, Smp. 215 ° (Zersetzung)

Beispiel 2

13.5 g (0.032 Mol) 2-{1-[3-(3-Chlor-2-pyrazinyloxy)-2-hydroxy-1-propyl]-4-piperidyl}-3,4-dihydro-1(2H)-isochinolon und 1.95 g (0.036 Mol) Natriummethylat in 150 ml Methanol werden während 10 Stunden unter Rühren am Rückfluss gekocht. Das Reaktionsgemisch wird im Wasserstrahlvakuum eingedampft. Der Rückstand wird in Essigester gelöst und mit 2n-Salzsäure ausgezogen. Die vereinigten salzsauren Extrakte werden mit konz. Natronlauge alkalisch gestellt und mit Methylenchlorid extrahiert. Die vereinigten Methylenchloridextrakte werden mit Wasser gewaschen, über Natriumsulfat getrocknet und im Wasserstrahlvakuum eingedampft. Der Rückstand kristallisiert aus Methanol/Aether.

Man erhält 8.2 g 2-{1-[3-(3-Methoxy-2-pyrazinyloxy)-2-hydroxy-1-propyl]-4-piperidyl}-3,4-dihydro-1(2H)-isochinolon, Smp. 122-124°.

Das mit methanolischer Salzsäure hergestellte Hydrochlorid kristallisiert aus Wasser/Aceton, Smp. 167-169°/190-193°.

Beispiel 3

12.5 g (0.03 Mol) 2-{1-[3-(3-Chlor-2-pyrazinyloxy)-2-hydroxy-1-propyl]-4-piperidyl}-3,4-dihydro-1(2H)-isochinolon, 2.78 g (0.045 Mol) Aethylmercaptan und 2.43 g (0.045 Mol) Natriummethylat in 200 ml Methanol werden während 10 Stunden unter Rühren am Rückfluss gekocht. Das Reaktionsgemisch wird im Wasserstrahlvakuum total eingedampft. Der Rückstand wird in Methylenchlorid gelöst und mit Wasser gewaschen. Die Methylenchloridlösung wird mit 2n-Salzsäure extrahiert. Die vereinigten salzsauren Extrakte werden mit konz. Natronlauge alkalisch gestellt und mit Methylenchlorid extrahiert. Die vereinigten Methylenchloridextrakte werden mit Wasser gewaschen, mit Natriumsulfat getrocknet und im Wasserstrahlvakuum eingedampft, wobei der Rückstand kristallisiert.

Man erhält 13 g 2-{1-[3-(2-Aethylthio-3-pyrazinyloxy)-2-hydroxy-1-propyl]-4-piperidyl}-3,4-dihydro-1(2H)-isochinolon, Smp. 136-138°.

Das mit methanolischer Salzsäure hergestellt Hydrochlorid kristallisiert aus Methanol/Aether, Smp. 200-202°.

Beispiel 4

5,0 g 1-[3-(3-Methoxy-2-pyrazinyloxy)-2-hydroxy-1-propyl]-4-(3,4-dihydro-1(2H)-2-isochinolonyl)-piperidinium-bromid werden in einem Gemisch von 30 ml Wasser und 30 ml Aethanol mit 2,0 g Platinoxyd bei 40° hydriert. Dann wird der Katalysator abfiltriert, das Filtrat im Vakuum eingedampft, zum Rückstand 2-n Natronlauge gegeben und die alkalische Phase mit Essigester ausgezogen, getrocknet, verdampft und aus Methanol/Aether umkristallisiert. Das 2-

{1-[3-(3-Methoxy-2-pyrazinyloxy)-2-hydroxy-1-propyl]-4-piperidyl} -3,4-dihydro-1(2H)-isochinolon hat einen Smp. von 122-124°. Das quaternäre Ausgangsmaterial wird durch Erhitzen von 3-(3-Methoxy-2-pyrazinyloxy)-2-hydroxy-1-brompropan mit 2-(4-Piperidinyl)-3,4-dihydro-1(2H)-isochinolon in Dimethylformamid bei 100° hergestellt.

Beispiel 5

44,9 g 1-[3-(3-Methoxy-2-pyrazinyloxy)-2-hydroxy-1-propyl]-4-[2-(2-chloräthyl)-benzoylamino]-piperidin werden in 200 ml Dimethylformamid gelöst und langsam dazu 90 ml 30%-ige Natriummethylatlösung getropft. Hierauf wird während 3 Stunden auf 50° erhitzt. Nach Verdampfen des Dimethylformamids im Vakuum wird der Rückstand in Essigester gelöst und die organische Phase mit 2-n Salzsäure ausgezogen. Die vereinigten salzsauren Extrakte werden mit konz. Natronlauge alkalisch gestellt und mit Methylenchlorid extrahiert. Die vereinigten Methylenextrakte werden mit Wasser gewaschen, mit Natriumsulfat getrocknet und im Vakuum eingedampft. Der Rückstand wird aus Methanol/Aether umkristallisiert. Das erhaltene 2-{1-[3-(3-Methoxy-2-pyrazinyloxy)-2-hydroxy-1-propyl]-4-piperidyl} -3,4-dihydro-1(2H)-isochinolon hat einen Smp. von 122-124°. Das Ausgangsmaterial wird durch Acylierung von 1-[3-(3-Methoxy-2-pyrazinyloxy)-2-hydroxy-1-propyl]-4-amino-piperidin mit 2-(2-Chloräthyl)-benzoylchlorid in Aceton unter Zusatz von Triäthylamin erhalten.

Patentansprüche

1. Piperidine der Formel

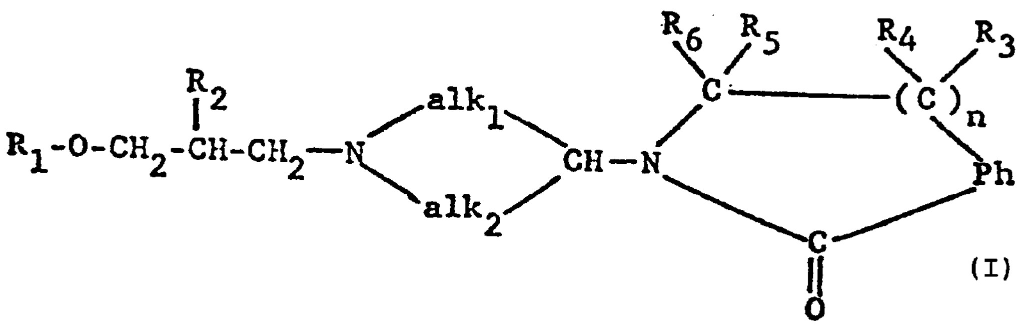

(I),

worin $R_1$ einen gegebenenfalls substituierten Heteroarylrest bedeutet, $alk_1$ und $alk_2$ unabhänig voneinander niedere Alkylenreste sind, die jeweils das mit ihnen verbundene Stickstoffatom und die mit ihnen verbundene Methingruppe durch 2 Kohlenstoffatome trennen, $R_2$ eine gegebenenfalls acylierte Hydroxylgruppe bedeutet, Ph einen gegebenenfalls substituierten o-Phenylenrest bedeutet, n = 0 oder 1 bedeutet, $R_3$ ein Wasserstoffatom, einen neideren Alkylrest oder die Hydroxylgruppe und $R_5$ einen niederen Alkylrest oder ein Wasserstoffatom bedeutet oder $R_3$ und $R_5$ zusammen für eine zweite Bindung stehen und $R_4$ und $R_6$ je ein Wasserstoffatom bedeuten, oder $R_4$ zusammen mit $R_3$ für eine Oxogruppe steht, $R_5$ einen niederen Alkylrest oder ein Wasserstoffatom und $R_6$ ein Wasserstoffatom bedeutet, oder $R_6$ zusammen mit $R_5$ für eine Oxogruppe steht, $R_3$ ein Wasserstoffatom, einen niederen Alkylrest oder die Hydroxylgruppe bedeutet und $R_4$ für ein Wasserstoffatom steht und deren Salze.

2. Verbindungen der Formel Ia

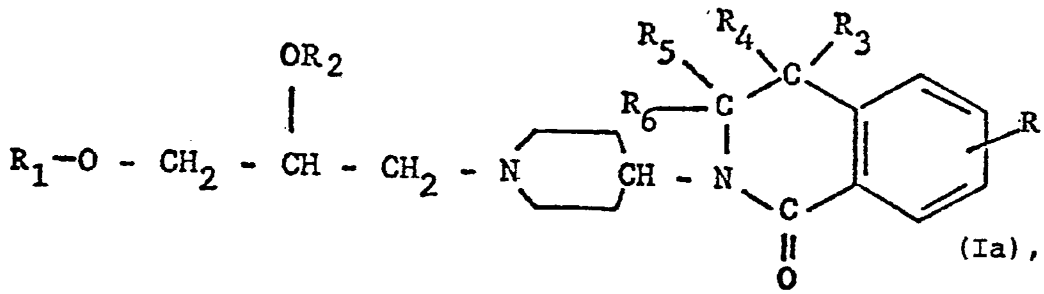

(Ia),

worin $R_1$ gegebenenfalls substituiertes, monocyclisches Heteroaryl oder Benzoheteroaryl mit 5 bis 6 Ringgliedern und 1 oder 2 Ringstickstoffatomen bedeutet, wobei Substituenten des heterocyclischen Arylrestes gegebenenfalls substituiertes Niederalkyl, z.B. Niederalkyl, Niederalkoxyniederalkyl, Niederalkanoylaminoniederalkyl oder Niederalkoxycarbonylaminoniederalkyl, oder gegebenenfalls veräthertes oder verestertes Hydroxy oder Mercapto, z.B. Niederalkoxy, Niederalkoxyniederalkoxy, Niederalkylthioniederalkoxy, Niederalkylthio oder Halogen sein können, $R_2$ einen niederen Alkanoylrest, wie insbesondere den Acetyl-, Propionyl- oder Pivaloylrest, oder vor allem ein Wasserstoffatom bedeutet, $R_3$ ein Wasserstoffatom oder die Hydroxylgruppe und $R_5$ ein Wasserstoffatom bedeutet oder $R_3$ und $R_5$ zusammen für eine zweite Bindung stehen und $R_4$ und $R_6$ je ein Wasserstoffatom bedeuten oder $R_4$ zusammen mit $R_3$ für eine Oxogruppe steht und $R_5$ und $R_6$ je ein Wasserstoffatom bedeuten oder $R_6$ zusammen mit $R_5$ für eine Oxogruppe steht und $R_3$ und $R_4$ je ein Wasserstoff bedeuten, und R einen Niederalkanoylaminorest, die Aminogruppe, die Nitrogruppe oder vor allem einen niederen Alkylrest, eine niedere Alkoxygruppe, ein Halogenatom, den Trifluormethylrest, die Hydroxylgruppe oder insbesondere ein Wasserstoffatom bedeutet, und ihre Salze.

3. Verbindungen der Formel Ia, worin $R_1$ gegebenenfalls durch Niederalkyl, z.B. Methyl, Niederalkoxy, z.B. Methoxy,

Niederalkylthio, z.B. Methylthio oder Aethylthio, und/oder Halogen mit Atomnummer bis zu 35, z.B. Chlor oder Brom, substituiertes monocyclisches Monoazaaryl oder Diazaaryl mit sechs Ringgliedern, wie Pyridyl, z.B. 2-, 3- oder 4-Pyridyl, Pyrimidinyl, z.B. 2- oder 4-Pyrimidinyl, oder Pyrazinyl, z.B. 2-Pyrazinyl, darstellt, $R_2$ den Acetyl-, Propionyl- oder insbesondere Pivaloylrest oder vor allem ein Wasserstoffatom bedeutet, $R_3$ ein Wasserstoffatom oder die Hydroxylgruppe und $R_5$ ein Wasserstoffatom bedeutet oder $R_3$ und $R_5$ zusammen für eine zweite Bindung stehen und $R_4$ und $R_6$ je ein Wasserstoffatom bedeuten oder $R_4$ zusammen mit $R_3$ für eine Oxogruppe steht und $R_5$ und $R_6$ je ein Wasserstoff bedeuten oder $R_6$ zusammen mit $R_5$ für eine Oxogruppe steht und $R_3$ und $R_4$ je ein Wasserstoffatom bedeuten, wobei vor allem $R_4$ und $R_6$ je für Wasserstoff stehen und $R_3$ und $R_5$ entweder für eine zweite Bindung stehen oder je ein Wasserstoffatom bedeuten, und R einen niederen Alkylrest, z.B. Methyl, einen niederen Alkoxyrest, z.B. Methoxy, ein Halogenatom, z.B. Chlor, den Trifluormethylrest oder insbesondere ein Wasserstoffatom bedeutet, und ihre Salze.

4. Verbindungen der Formel Ia, worin $R_1$ für Pyrazinyl, ferner für Pyridyl, z.B. 2- oder 3-Pyridyl steht, das in ortho-Stellung zum Verknüpfungskohlenstoffatom vorzugsweise durch Niederalkyl, z.B. Methyl, Niederalkoxy, z.B. Methoxy, Niederalkylthio, z.B. Methylthio oder Aethylthio, oder Halogen mit Atomnummer bis zu 35, z.B. Chlor oder Brom, substituiert ist, und gegebenenfalls weitere Substituenten dieser Art enthalten kann, und $R_2$, $R_3$, $R_4$, $R_5$, $R_6$ und R Wasserstoff bedeuten, und Salze, insbesondere Säureadditonssalze, in erster Linie pharmazeutisch verwendbare, nichttoxische Säureadditionssalze davon.

5. Verbindungen der Formel Ia, die als $R_1$ einen vorzugsweise substituierten 2-Pyrazinylrest aufweisen, und $R_2$, $R_3$,

$R_4$, $R_5$, $R_6$ und R Wasserstoff bedeuten, und ihre nicht-toxischen Säureadditionssalze.

6. 2-[1-[3- (3-Methoxy-2-pyrazinyloxy)-2-hydroxy-1-propyl]-4-piperidyl]-3,4-dihydro-1(2H)-isochinolon und seine therapeutisch verwendbaren Salze.

7. Verbindungen der Formel Ib

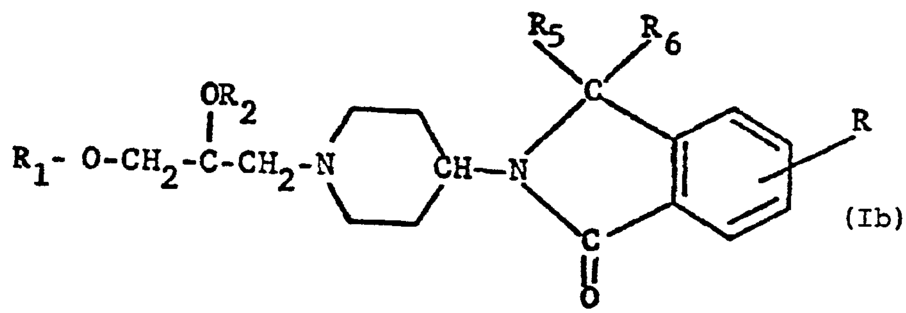

(Ib),

worin $R_1$ gegebenenfalls substituiertes, monocyclisches Heteroaryl oder Benzoheteroaryl mit 5 bis 6 Ringgliedern und 1 oder 2 Ringstickstoffatomen im Rest $R_1$ bedeutet, wobei Substituenten des heterocyclischen Arylrestes gegebenenfalls substituiertes Niederalkyl, z.B. Niederalkyl, Niederalkoxyniederalkyl, Niederalkanoylaminoniederalkyl oder Niederalkoxycarbonylaminoniederalkyl, oder gegebenenfalls veräthertes oder verestertes Hydroxy oder Mercapto, z.B. Niederalkoxy, Niederalkoxyniederalkoxy, Niederalkylthioniederalkoxy, Niederalkylthio oder Halogen,
R je ein Wasserstoffatom, einen niederen Alkylrest, eine Hydroxygruppe, einen niederen Alkoxyrest, ein Halogenatom, eine Trifluormethylgruppe, die Nitrogruppe, eine Aminogruppe, eine Alkanoylaminogruppe, $R_2$ ein Wasserstoffatom oder eine niedere Alkanoylgruppe, insbesondere den Acetyl-, Propionyl- oder Pivaloylrest, $R_5$ und $R_6$ je ein Wasserstoffatom, oder $R_5$ und $R_6$ zusammengenommen eine Oxogruppe bedeuten, und ihre Salze.

8. Verbindungen der Formel Ib, worin $R_1$ für gegebenenfalls durch Niederalkyl, z.B. durch Methyl, Niederalkoxy, z.B. Methoxy, Niederalkylthio, z.B. Methylthio oder Aethylthio, und/oder Halogen mit Atomnummer bis zu 35, z.B. Chlor oder Brom, substituiertes 2-Pyrazinyl, sowie für gegebenenfalls entsprechend substituiertes Pyridyl, z.B. 2- oder 3-Pyridyl steht, wobei Substituenten irgendeine Stellung, mindestens einer jedoch vorzugsweise die ortho-Stellung zum Verknüpfungsringkohlenstoffatom des Heteroarylrestes einnehmen können, R je ein Wasserstoffatom, die Methyl-, Methoxy- oder Acetylaminogruppe, $R_2$ ein Wasserstoffatom, die Acetyl-, Propionyl- oder die Pivaloylgruppe, $R_5$ und $R_6$ je ein Wasserstoffatom bedeuten, und ihre Salze.

9. Verbindungen der Formel Ib, worin $R_1$ gegebenenfalls durch Niederalkyl, z.B. Methyl, Niederalkoxy, z.B. Methoxy, Niederalkylthio, z.B. Methylthio oder Aethylthio, und/oder Halogen mit Atomnummer bis zu 35, z.B. Chlor oder Brom, substituiertes monocyclisches Monoazaaryl oder Diazaaryl mit sechs Ringgliedern, wie Pyridyl, z.B. 2-, 3- oder 4-Pyridyl, Pyrimidinyl, z.B. 2- oder 4-Pyrimidinyl, oder Pyrazinyl, z.B. 2-Pyrazinyl darstellt, und $R_2$ ein Wasserstoffatom, die Acetyl-, Propionyl- oder die Pivaloylgruppe und R, $R_5$ und $R_6$ je ein Wasserstoffatom bedeuten, und ihre nicht-toxischen Säureadditionssalze.

10. Verbindungen der Formel Ib, worin $R_1$ für 2-Pyrazinyl, ferner für Pyridyl, z.B. 2- oder 3-Pyridyl steht, das in ortho-Stellung zum Verknüpfungskohlenstoffatom vorzugsweise durch Niederalkyl, z.B. Methyl, Niederalkoxy, z.B. Methoxy, Niederalkylthio, z.B. Methylthio oder Aethylthio, oder Halogen mit Atomnummer bis zu 35, z.B. Chlor oder Brom, substituiert ist, und gegebenenfalls weitere Substituenten dieser Art enthalten kann, und R, $R_2$, $R_5$ und $R_6$ je ein Wasserstoffatom bedeuten,

und ihre nicht-toxischen Säureadditionssalze.

11. Pharmazeutische Präparate enthaltend eine der Verbindungen der Ansprüche 1 bis 10.

12. Die Verbindungen der Ansprüche 1 bis 10 zur Verwendung als Pharmazeutika.

13. Die Verbindungen der Ansprüche 1 bis 10 zur Verwendung als Antihypertensiva.

14. Verfahren zur Herstellung neuer Piperidine der Formel I

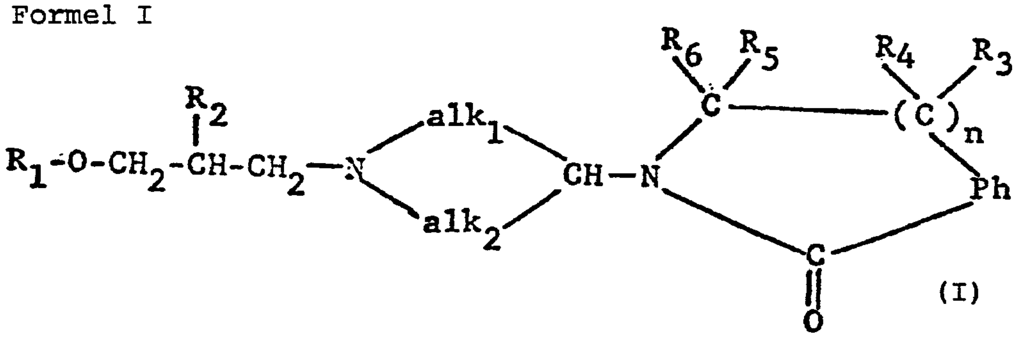

(I)

worin $R_1$ einen gegebenenfalls substituierten Heteroarylrest bedeutet, $alk_1$ und $alk_2$ unabhängig voneinander niedere Alkylenreste sind, die jeweils das mit ihnen verbundene Stickstoffatom und die mit ihnen verbundene Methingruppe durch 2 Kohlenstoffatome trennen, $R_2$ eine gegebenenfalls acylierte Hydroxylgruppe bedeutet, Ph einen gegebenenfalls substituierten o-Phenylenrest bedeutet, n = 0 oder 1 bedeutet, $R_3$ ein Wasserstoffatom, einen niederen Alkylrest oder die Hydroxylgruppe und $R_5$ einen niederen Alkylrest oder e der ein Wasserstoffatom bedeutet oder $R_3$ und $R_5$ zusammen für eine zweite Bindung stehen und $R_4$ und $R_6$ je ein Wasserstoffatom bedeuten, oder $R_4$ zusammen mit $R_3$ für eine Oxogruppe steht, $R_5$ einen niederen Alkylrest oder ein Wasserstoffatom und $R_6$ ein Wasserstoffatom bedeutet, oder $R_6$ zusammen mit $R_5$ für eine Oxogruppe steht, $R_3$ ein Wasserstoffatom, einen niederen Alkylrest oder die Hydroxyl-

gruppe bedeutet und $R_4$ für ein Wasserstoffatom steht, und deren Salze, dadurch gekennzeichnet, dass man

a) eine Verbindung der Formel

$$R_1 - O - Y_1$$

mit einer Verbindung der Formel

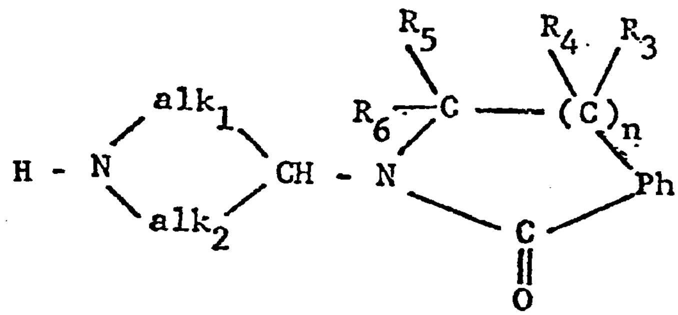

(III),

umsetzt, wobei $R_1$, $alk_1$, $alk_2$, Ph, n, $R_3$, $R_4$, $R_5$ und $R_6$ die angegebenen Bedeutungen haben, $Y_1$ für einen Rest der Formel

$$- CH_2 - \underset{X}{\underset{|}{CH}} - CH_2 - Z$$

und X für die Gruppe $R_2$, wobei $R_2$ die angegebene Bedeutung hat, steht und Z eine reaktionsfähige veresterte Hydroxylgruppe bedeutet, oder X und Z zusammen eine Epoxygruppe bilden, oder

b) eine Verbindung der Formel

$$R_1 - OH \qquad \text{(IIa)},$$

worin $R_1$ obige Bedeutung hat, mit einer Verbindung der Formel

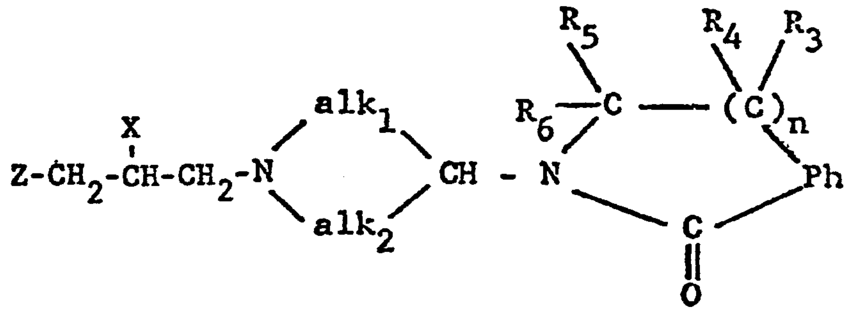

(IIa)

worin X, Z, $alk_1$, $alk_2$, Ph, n, $R_3$, $R_4$, $R_5$ und $R_6$ obige Bedeutungen haben, umsetzt, oder

c) in einer Verbindung der Formel

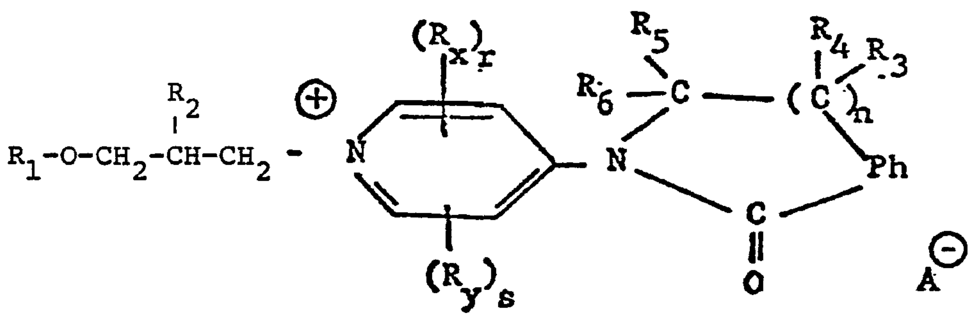

(V),

worin $R_1$, $R_2$, $R_3$, $R_4$, $R_5$, $R_6$, Ph und n obige Bedeutungen haben, $R_x$ und $R_y$ unabhängig voneinander niedere Alkylreste oder Wasserstoffatome bedeuten und r uns s für 1 oder 2 stehen, und $A^{\ominus}$ ein Anion bedeutet, den Pyridiniumring zum Piperidinring reduziert, oder

d) eine Verbindung der Formel

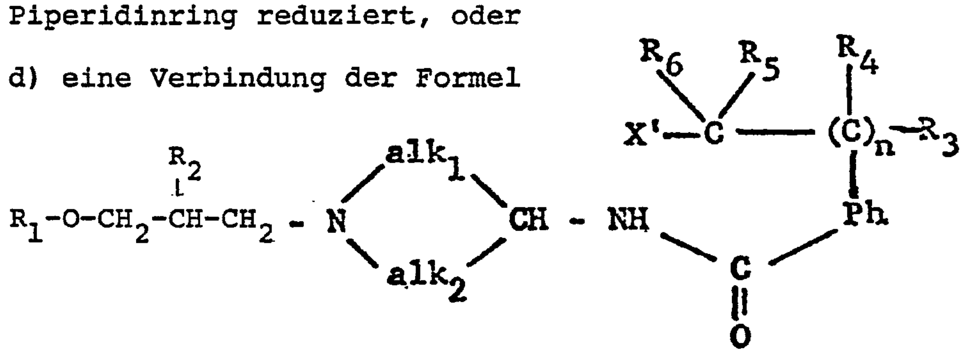

(VI),

worin $R_1$, $R_2$, $alk_1$, $alk_2$ Ph, n, $R_3$, $R_4$, $R_5$ und $R_6$ die angegebenen Bedeutungen haben und X' eine reaktionsfähige veresterte Hydroxylgruppe bedeutet, intramolekular kondensiert, oder

e) für Verbindungen der Formel I, worin $R_3$ und $R_5$ zusammen für eine zweite Bindung stehen, wenn n = 1 ist eine Verbindung der Formel

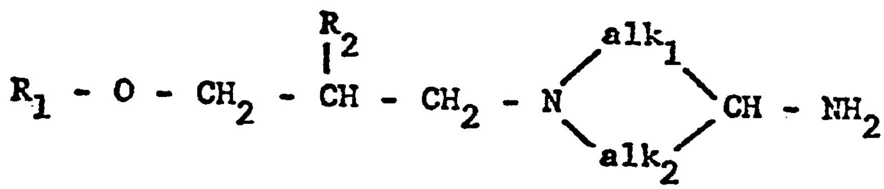

(VII),

mit einer Verbindung der Formel

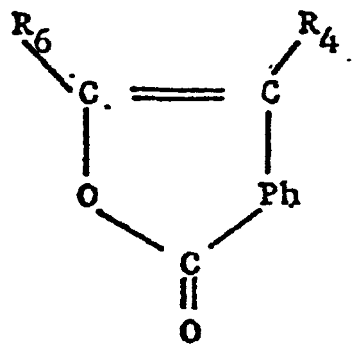

(VIII),

worin $R_1$, $R_2$, $R_4$, $R_6$, $alk_1$, $alk_2$ und Ph die angegebenen Bedeutungen haben, umsetzt, oder

f) eine Verbindung der Formel

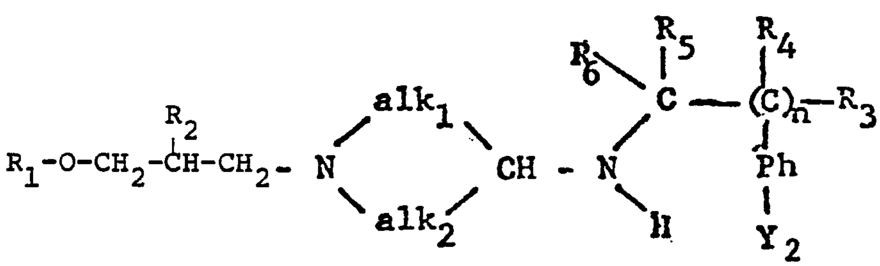

(IX),

worin $R_1$, $R_2$, $alk_1$, $alk_2$, $R_3$, $R_4$, $R_5$, $R_6$, n und Ph die angegebenen Bedeutungen haben und $Y_2$ eine freie oder vorzugsweise eine eine Oxogruppe enthaltende funktionell abgewandelte Carboxylgruppe bedeutet, intramolekular kondensiert,

oder

g) für Verbindungen der Formel I, worin $R_2$ für Wasserstoff geht, in einer Verbindung der Formel

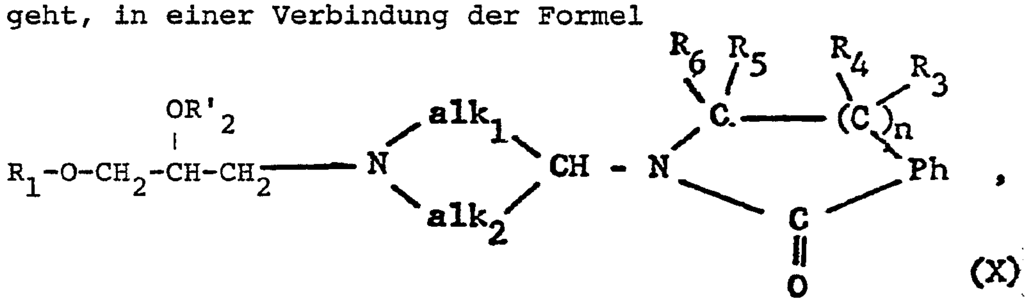

(X),

worin $R_1$, $alk_1$, $alk_2$, Ph, n, $R_3$, $R_4$, $R_5$ und $R_6$ die angegebenen Bedeutungen haben und $R'_2$ einen durch Hydrogenolyse abspaltbaren Rest bedeutet, $R'_2$ durch Hydrogenolyse abspaltet, oder

h) für Verbindungen der Formel I, worin $R_6$ zusammen mit $R_5$ eine Oxogruppe bildet, eine Verbindung der Formel

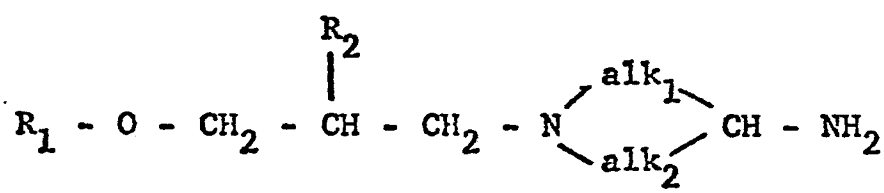

worin $R_1$, $R_2$ $alk_1$ und $alk_2$ obige Bedeutungen haben, mit einer Verbindung der Formel

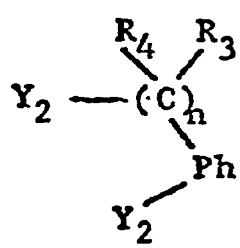

(XI),

worin $R_3$, $R_4$, n und Ph obige Bedeutungen haben und die Substituenten $Y_2$ unabhängig voneinander für eine freie, oder vorzugsweise eine eine Oxogruppe enthaltende funktionell abgewandelte Carboxylgruppe oder zusammen für die

Gruppierung

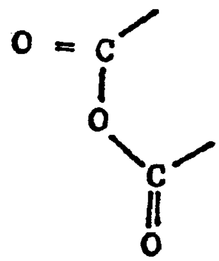

stehen, umsetzt, und, wenn erwünscht, in erhaltenen Verbindungen der Formel I im Rahmen der Definition der Endstoffe einen oder mehrere Substituenten einführt, abwandelt oder abspaltet und/oder ein erhaltenes Isomerengemisch in die reinen Isomeren auftrennt und/oder ein erhaltenes Racemat in die optischen Antipoden aufspaltet und/oder ein erhaltenes Salz in die freie Verbindung in eines ihrer Salze überführt.

15. Das in den Beispielen 1 bis 5 beschriebene Verfahren.

FO 7.4 HFO/rn

Europäisches Patentamt

# EUROPÄISCHER RECHERCHENBERICHT

Nummer der Anmeldung

EP 78 10 0491 / 0 000 869

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | DE - A - 2 533 567 (CIBA-GEIGY)<br>* Patentansprüche 15,47 *<br>--<br>BE - A - 850 556 (CIBA-GEIGY)<br>* Patentansprüche 1,4,13 *<br>---- | 1,11<br><br><br>1,11 | C 07 D 401/14<br>A 61 K 31/495<br>//(C 07 D 401/14<br>C 07 D 241/18<br>C 07 D 217/24<br>C 07 D 211/58)<br><br>RECHERCHIERTE SACHGEBIETE (Int. Cl.²)<br><br>C 07 D 401/14<br>A 61 K 31/495<br>//(C 07 D 401/14<br>C 07 D 241/18<br>C 07 D 217/24<br>C 07 D 211/58)<br><br>KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| [illegible] | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Pr'ifer |
|---|---|---|
| Den Haag | 30-10-1978 | ALFARO |